# EUROPEAN PATENT SPECIFICATION

(11) **EP 2 510 114 B1**
(45) Date of publication and mention of the grant of the patent: **20.04.2016**
(21) Application number: 10788340.7
(22) Date of filing: 10.12.2010
(51) Int. Cl.: C12Q 1/68

(54) **RNA ANALYTICS METHOD**
RNA-Analyseverfahren
Procédé analytique pour ARN

(30) Priority: 11.12.2009 EP 09178923
(43) Date of publication of application: 17.10.2012
(73) Proprietor: Lexogen GmbH, 1230 Vienna (AT)
(72) Inventor: SEITZ, Alexander, A-1140 Vienna (AT); PAUL, Lukas, A-1230 Vienna (AT); van MIN, Max Jan, NL-2497 ZJ Den Haag (NL)
(74) Representative: Sonn & Partner Patentanwälte
(86) International application number: PCT/EP2010/069382
(87) International publication number: WO 2011/070155

(56) References cited:
- WO-A2-99/55913
- WO-A2-2007/073171
- US-B1- 6 261 770
- NAGALAKSHMI UGRAPPA ET AL: "The transcriptional landscape of the yeast genome defined by RNA sequencing", SCIENCE (WASHINGTON D C), vol. 320, no. 5881, June 2008 (2008-06), pages 1344-1349, XP002573150, ISSN: 0036-8075
- ARMOUR CHRISTOPHER D ET AL: "Digital transcriptome profiling using selective hexamer priming for cDNA synthesis", NATURE METHODS, vol. 6, no. 9, September 2009 (2009-09), page 647, XP009125354, ISSN: 1548-7091
- BREYNE P ET AL: "Quantitative cDNA-AFLP analysis for genome-wide expression studies.", MGG MOLECULAR GENETICS AND GENOMICS, vol. 269, no. 2, May 2003 (2003-05), pages 173-179, XP002573151, ISSN: 1617-4615
- MATZ M ET AL: "ORDERED DIFFERENTIAL DISPLAY: A SIMPLE METHOD FOR SYSTEMATIC COMPARISON OF GENE EXPRESSION PROFILES", NUCLEIC ACIDS RESEARCH, OXFORD UNIVERSITY PRESS, SURREY, GB, vol. 25, no. 12, 1 January 1997 (1997-01-01), XP002911919, ISSN: 0305-1048
- WILHELM BRIAN T ET AL: "RNA-Seq-quantitative measurement of expression through massively parallel RNA-sequencing", METHODS (AMSTERDAM), vol. 48, no. 3, July 2009 (2009-07), pages 249-257, XP026284846, ISSN: 1046-2023
- FREDRIKSSON SIMON ET AL: "Multiplex amplification of all coding sequences within 10 cancer genes by Gene-Collector", NUCLEIC ACIDS RESEARCH, OXFORD UNIVERSITY PRESS, SURREY, GB, vol. 35, no. 7, 1 February 2007 (2007-02-01), pages e47.1-e47.6, XP002487873, ISSN: 0305-1048, DOI: 10.1093/NAR/GKM078
- CHEN WEI ET AL: "Mapping translocation breakpoints by next-generation sequencing", GENOME RESEARCH, vol. 18, no. 7, July 2008 (2008-07), pages 1143-1149, ISSN: 1088-9051
- PANSRI POTJAMAS ET AL: 'A compact phage display human scFv library for selection of antibodies to a wide variety of antigens' BMC BIOTECHNOLOGY, BIOMED CENTRAL LTD. LONDON, GB vol. 9, no. 1, 29 January 2009, pages 1 - 16, XP021049493 DOI: 10.1186/1472-6750-9-6 ISSN: 1472-6750
- SAHOTA SURINDER S ET AL: "Myeloma V-L and V-H gene sequences reveal a complementary imprint of antigen selection in tumor cells", BLOOD, vol. 89, no. 1, 1997, pages 219-226, XP007922684, ISSN: 0006-4971

## Description

The present invention relates to the field of analyzing complex mixtures of nucleic acids and sample preparation for characterization methods and sequencing, especially high throughput sequencing techniques, such as Next Generation Sequencing (NGS).

NGS is currently the foremost complete analyzing method. Next Generation Sequencing is a generic term for parallelized sequencing through polymerization as high-throughput DNA sequencing method. NGS reads sequences of up to many million fragments which are typically between 10 to several hundred base-pairs long. The complete sequence is obtained by alignment of those reads which is a challenging task. Some NGS methods rely on a consensus blue print held in genomic and/or transcriptomic databases. The quality of the results depends on length and number of reads, reading accuracy, quality of information in the reference database and applied bioinformatics algorithms. To date many reads provide just limited information. For instance many of the reads cannot be assigned uniquely and therefore are discarded. The two basic underlying reasons for this assignment uncertainty is that a) one read can align with two or more genes and b) that one read can originate from different tran-scriptvariants of the same gene.

In addition, the sequencing depth and therefore the detection of low abundant nucleic acids is limited. For the analysis of RNA this implies that in samples, which contain a multitude of different RNA molecules of different cells or cell populations or disease organisms, rare RNA or parts thereof are less likely to be retrieved. In fact, in transcriptomics rare RNA transcripts of even a simple organism are less likely to be detected and quantified.

In more detail, for generating detectable signals most NGS approaches must amplify individual RNA molecules or their DNA copy. Emulsion polymerase chain reaction (PCR) isolates individual DNA molecules using primer-coated beads in aqueous bubbles within an oil phase. Singularizing of DNA molecules, e.g. by rigorous dilution is another option. Another method for in vitro clonal amplification is bridge PCR, where fragments are amplified upon primers attached to a solid surface. Another option is to skip this amplification step, directly fixing DNA molecules to a surface. Such DNA molecules or above mentioned DNA coated beads are immobilized to a surface, and sequenced in parallel. Sequencing by synthesis, like the "old style" dye-termination electrophoretic sequencing, uses a DNA polymerase to determine the base sequence. Reversible terminator methods use reversible versions of dye-terminators, adding one nucleotide at a time, detecting fluorescence at each position, by repeated removal of the blocking group to allow polymerization of another nucleotide. Pyrosequencing also uses DNA polymerization, adding one nucleotide species at a time and detecting and quantifying the number of nucleotides added to a given location through the light emitted by the release of attached pyrophosphates. The sequencing by ligation method uses a DNA ligase to determine the target sequence. Used in the polony method and in the SOLiD^{®} technology, it employs a partition of all possible oligonucleotides of a fixed length, labeled according to the sequenced position. Oligonucleotides are annealed and ligated. The preferential ligation by DNA ligase for matching sequences results in a dinucleotide encoded colour space signal at that position.

NGS technologies are essentially based on random amplification of input DNA. This simplifies preparation but the sequencing remains undirected. The sheer complexity of the sample information - simultaneously obtained - is the key hindrance for unambiguous alignment of the reads. Therefore, complexity reduction is essential for increasing the quality of the results.

The classical route for DNA complexity reduction, e.g. employed during the human genome project, is to create BAC (bacterial artificial chromosome) clones prior to sequencing. Distinct stretches of genomic DNA are cloned into bacterial host cells, amplified, extracted and used as templates for Sanger sequencing. Production, maintenance and verification of large BAC libraries are laborious processes and associated with appreciable costs. Due to these impracticalities and the incompatibility with existing NGS platforms it is generally sought to avoid bacterial cloning.

Another option to reduce complexity is to first select polynucleic acids based on their respective sizes. Different approaches include, but are not limited to, agarose gel electrophoresis or size exclusion chromatography for fractionation. Small RNA sequencing approaches employ this method in order to obtain e.g. a fraction of RNA molecules called micro RNA (miRNA) sized between 15 and 30 nucleotides.

The probably most straightforward approach of complexity reduction is by limiting the amount of input nucleic sample to a single cell. Single-cell sequencing approaches rely on amplification reactions from highly dilute solutions, are incapable of actually reducing the complexity inherent to cellular content since it contains an entire transcriptome, and are based solely on a selection of the input cells.

A different method for reducing the amount of input nucleic acid to below the amount contained within a single cell sometimes is termed limited dilution. A genomic nucleic acid sample is first fragmented and then diluted to an extent where spatial distribution of the nucleic acid fragments within the sample volume becomes significant. Then subpools are created by taking such small volumes from the total sample volume that most subpools contain no nucleic acids, a few subpools contain one nucleic acid each and even less subpools contain two nucleic acids. This leads to singularization of nucleic acids and therefore to complexity reduction compared to the full length genome as each singularized nucleic acid is a fragment of a genome. Therefore an increased sequence assembly efficiency for the individual nucleic acid fragments containing sub-pools is gained. Assembly and scaffold building for large genomes thereby is facilitated. In transcriptome analysis such a limited dilution approach will not reduce the complexity introduced through variations in expression of the same gene or different genes as each transcript molecule will occupy one subpool and therefore as many subpools are needed as molecules in the sample to display the entire transcriptome of a sample.

A further option is to sequence-specifically reject RNA, e.g. in a hybridization-based approach that removes ribosomal RNA from the entire RNA sample. As opposed to other fractionation methods that rely either on prior sequence information or are directed towards a certain RNA fraction (e.g. polyA selection), removal of rRNA does not bias the sequencing sample if e.g. mRNA is investigated. Methods that deplete rRNA from total RNA samples are used to increase the number of reads that cover mRNA and other transcripts. However,the complexity of read al-lignemt to a certain gene or transcripts of a gene is only not reduced.

It is also possible to employ sequence-specific selection methods, e.g. by targeted sequencing of genomic regions such as particular exons. The idea behind such capture arrays is to insert a selection step prior to sequencing. Those arrays are programmed to capture only the genomic regions of interest and thus enabling users to utilize the full capacity of the NGS machines in the sequencing of the specific genomic regions of interest. Low density, on array capture hybridization is used for sequencing approaches. Such technology is not "hypothesis neutral", as specific sequence information is required for the selection process.

A similar positive selection can be used for targeted re-sequencing. E.g. biotinylated RNA strands of high specificity for their complementary genomic targets can be used to extract DNA fragments for subsequent amplification and sequence determination. This form of complexity reduction is necessarily based on available sequence information and therefore not hypothesis neutral.

Preparations of genomes that reduce the complexity of the sample have been disclosed in WO 2006/137734 and WO 2007/073171 A2. They are based on AFLP technology (EP 0534858 and Breyne et al. (MGG Mol. Genet. Genom., 269 (2) (2003): 173-179)). AFLP has also been applied to double stranded cDNA that is derived from RNA. Here the double stranded cDNA is first cut by a restriction enzyme and then fragments are segregated. Even though the complexity of the nucleic acid fragments contained in each subpool decreases, in the majority of the cases each fragment of a nucleic acid will be segregated into at least two different sub-pools.
This means e.g. that the subpool information cannot be used for assembly of the nucleic acids of the sample after sequencing, as likely each restriction fragment of a nucleic acid is in a different subpool. Therefore when restricting cDNA during cDNA AFLP information towards the full length of the cDNA gets lost. In essence methods such as AFLP that fragment the sample before segregation do not reduce complexity in terms of alignment of full length transcript sequences. This ambiguity is further increased as for covering the sequence of most cDNAs with at least one restriction site a multitude of restriction enzymes must be used.
In addition the transcriptome is only statistically covered in a cDNA AFLP approach as the pool of restriction enzymes may or may not cut a nucleic acid.

In Differential Display (Liang 1992, Matz 1997) only partial sequences of mRNA or its cDNAs are represented and therefore again no full length sequences can be assembled nor can reads be assigned to transcript variants of a gene that share the same 3' sequence.

Sequencing of 16S rDNA or 16S rRNA sequences from mixed samples of microorganisms is in general employed for detection of rare species within these samples. By restricting the sequencing approach to a specific signature of microorganisms both complexity and information content are reduced. Frequently only phylogenetic information is obtained.

Tag-based identification of transcripts includes SAGE (Serial Analysis of Gene Expression) wherein sequence tags of defined length are extracted and sequenced. Since the initial creation of tag concatemers is a disadvantage for NGS, derived protocols are used omitting this step.

A related method is CAGE (Cap Analysis of Gene Expression). CAGE is intended to yield information on the 5' ends of transcripts and therefore on their respective transcription start sites. 5' cap carrying RNA molecules are selected before end-tags are extracted and sequenced.

Although only defined parts of the transcriptome are extracted for analysis SAGE and CAGE have their limitations because they do not allow for comprehensive segregation.

Nagalakshmi et al. (Science, 320 (5881) (2008): 1344-1349) and Wilhelm et al. (Methods, 48 (3) (2009): 249-257) relate to the RNA-Seq method comprising generation of a cDNA by using a poly-A and a random hexamer primer. This method does not allow the reduction of complexity for assigning reads to individual transcript variants.

Armour et al. (Nature Methods, 6 (9) (2009): 647) relates to the generation of a cDNA from an RNA pool for sequencing. By using so called "not-so-random" (NSR) primers rRNA is depleted. In this method only short sequence fragments are segregated. This method therefore does not reduce complexity of full length transcripts.

Therefore there is a need for methods that can provide for smaller fractions of a nucleic acid sample and provide for means to improve the sequencing or detection procedure, in particular for improving detection of rare nucleic acid samples e.g. in pools of nucleic acids of high concentrations, which reduce the chance to obtain signals of rare nucleic acids.

Therefore the present invention provides a method of ordering nucleic acid molecule fragment sequences derived from a pool of potentially diverse RNA molecules comprising
- optionally reverse transcribing the RNA molecules to provide a pool of cDNA molecules,
- segregating nucleic acids from said template RNA or cDNA pool by selecting for potentially different templates with a distinctive nucleic acid feature shared by the segregated templates, comprising selecting for potentially different templates with at least one given nucleotide type shared by the segregated templates at a certain position being i) within 100 nucleotides from either the 5' or 3' terminus of the full length template nucleic acid molecule sequence or ii) selected from the 1 to 100 nucleotides next to a polyA-tail or next to a polyT-tail of a cDNA or iii) selected from the 1 to 100 nucleotides next to a tail artificially attached to the template RNA or cDNA, and wherein said selecting is with a primer or probe, which is specific for said at least one distinctive nucleotide i), ii) or iii), thereby providing at least a first subpool of nucleic acids,
- optionally once or more further segregating nucleic acids from said template RNA or cDNA, selectively segregating nucleic acids with a different distinctive nucleic acid feature, thereby providing one or more further subpool(s) of nucleic acids,
- generating fragments of said segregated nucleic acid molecules by
   a) random fragmenting or
   b) obtaining fragment copies of said segregated nucleic acid molecules,
- attaching linkers or adaptors to the generated fragments, wherein the fragments of each subpool or combined subpools remain separable from fragments of other subpools or other combined subpools by physically separating the subpools or by attaching a label to the fragments of the subpools, with the label identifying a subpool.

Also provided is a method of ordering nucleic acid molecule fragment sequences derived from a pool of potentially diverse RNA molecules com-prising
· optionally reverse transcribing the RNA molecules to pro-vide a pool of cDNA molecules,
· segregating nucleic acids from said template RNA or cDNA pool by selecting for potentially different templates with a distinctive nucleic acid feature shared by the segregated tem-plates, comprising selecting for potentially different templates with at least one given nucleotide type shared by the segregated templates at a certain position being i) within 100 nucleotides from either the 5' or 3' terminus of the full length template nucleic acid molecule sequence or ii) selected from the 1 to 100 nucleotides next to a polyA-tail or next to a polyT-tail of a cDNA or iii) selected from the 1 to 100 nucleotides next to a tail artificially attached to the template RNA or cDNA, and wherein said selecting is with a primer or probe, which is spe-cific for said at least one distinctive nucleotide i), ii) or iii), thereby providing at least a first subpool of nucleic ac-ids,
· optionally once or more further segregating nucleic acids from said template RNA or cDNA, selectively segregating nucleic acids with a different distinctive nucleic acid feature, thereby providing one or more further subpool(s) of nucleic acids,
· generating fragments of said segregated nucleic acid molecules by determining a partial sequence of said segregated nu-cleic acid molecule by a nano-pore.

The present invention is further defined in the claims.

The inventive segregation step has the advantage that subpools of nucleic acids are provided and this subpool information can be used to improve further sequencing reactions, e.g. Next Generation Sequencing which is based on obtaining reads of small fragments of the nucleic acids or other nucleic acid characterization methods. It is possible with the inventive method that the subpool information can accompany the nucleic acids and the fragments and this information is used for alignment of the sequencing reads and the determination of the concentration of an individual nucleic acid sequence within the subpool. Furthermore, subpooling can reduce complexity to such a degree that transcripts of an organism and/or transcripts of different cells or cell populations and/or transcripts of different organisms that are present in a sample in different concentrations can be segregated in order to increase the likelihood of detecting rare nucleic acids within the sample of abundant RNA entities. Furthermore, it allows the detection and identification of sequencing reads belonging to different transcript variants, such as splice variants.

For unambiguous alignment of sequencing reads and the subsequent precise sequence assembly, efficient procedures for the reduction of sample complexity are required. A high degree of the complexity of the original material results from its disorder, the blend of sequences of different concentrations. Some advantages the inventive methods can provide are segregation methods which can
i) provide defined sub-pools of nucleic acid samples with common characteristics,
ii) provide means for coupling the sub-pool specific information to the nucleic acids and fragments thereof, and
iii) facilitate the concentration measurement of individual sequences within the sub-pool and consequently within the original sample,
to improve the quality of sequencing reads alignment and/or to analyze the original sample by other means.

With this method it is possible to reduce the complexity of transcriptomic samples to such a degree that rare transcripts can be detected within the main competing signal of all other, possibly highly abundant transcripts. The method is suitable to measure quantitatively sequences and fragments thereof from the very rare to the highly abundant forms.

The core of this invention is the sorting of a nucleic acid pool into sub-pools prior the fragmentation step (e.g. required by NGS), where all nucleic acids fragments acquire the additional sub-pool information of their parental molecule. This information can be maintained throughout the sequence reading, e.g. partial sequence determination. Then, every read contains the sequence and the sub-pool information, which provides main advantages during the read alignment procedures. One has to solve parallel just several smaller instead of one large "puzzle". The complexity of the task becomes significantly reduced. As a result, i) multi-position assignments are more unlikely, ii) the origin of more reads can be determined which have been formerly classified with "no-match", iii) in the case of transcript analysis, splice junctions and transcription start site variation are detected with higher probability, and iv) more full-length transcripts can be detected.

The sub-pooling of transcript pools can be achieved through sub-pools with different additional information content. The gained benefits depend on the chosen methods.

Segregation into subpools can be performed by exploiting transcript properties as distinctive nucleic acid feature which are directly or indirectly sequence related. Such properties are for example the affinity to adsorbing matters like various column materials (e.g. silica gel) or the solubility in the presence of salts, polymers or other additives. In such indirect sequence related segregation the required information on the sample nucleic acids is limited, e.g. precipitation depends predominantly on length, the GC-content and secondary structures. The distinctive nucleic acid feature can be an adsorption or solubility property.

Sub-pools can be generated through methods which utilize distinctive sequence information like i) partial internal or terminal sequences or/and ii) transcript size.
i) Using distinctive sequences (usually small nucleotide sequence portions) is the most powerful segregation tool. E.g. a distictive nucleic acid feature can be a partial sequence of the nucleic acids stemming from the template RNA or cDNA. The distinctive sequence can be a single nucleotide type (e.g selected from A, T, U, G or C) or more at a specific position within the nucleic acids to be segregated. E.g. nucleotides can be segregated for the presence of one or more nucleotide types or sequences at either the 5' or 3' terminus or in a given distance from said terminus. On one hand an array of hybridization probes, which covers one or more sequence possibilities of said distinctive portion of the nucleic acid, can be used to create sub-pools. Even if sub-pools contain different nucleic acids and some nucleic acids will be present in several sub-pools, such segregation approach already reduces the complexity of the original pool. After collecting all reads it is known to the aligment algorithm that the transcripts contain the subpool specific sequence(s), preferably the alignment algorithm must ensure, that all transcripts display at least one sub-pool specific sequence.
   Segregation by selecting for a distinctive nucleic acid feature like a distinctive sequence (e.g. a single nucleotide or partial sequence at a specific position as described above) can be performed by either selecting such nucleic acids with the distinctive sequence or by specifically amplifying nucleic acids with said distinctive sequence and further utilising these amplicons in the inventive method.
   A preferred segregation method uses the sequence information of both termini, thus start and end site of the nucleic acids. After termini-specific amplification and if the redundancy in the sequence specificity is zero (no mismatch allowed), then all sub-pools contain amplicons, e.g. PCR products, with exactly those termini. Hence, sub-pools can contain several nucleic acids of RNA molecules such as transcripts, but each nucleic acid is only presented in one sub-pool. By this means, the complexity of the alignment procedure is largely reduced.
ii) The RNA molecule size can be exploited to segregate the RNA according to the number of nucleotides per RNA via electrophoresis techniques (gel or capillary electrophoresis), or other methods. The later alignment of the different reads per sub-pool can benefit from the boundary condition of a certain rather narrow size range.

As used herein nucleic acid molecule derived from an RNA molecule refers to a nucleic acid of any type with the same sequence as the RNA from the sample.

In particular preferred during the segregation step full length or complete nucleic acids are segregated or selected from the template RNA or cDNA pool. Segregation of full length or complete nucleic acids at this step, prior to fragmenting, has the benefit that each segregated pool contains the sequence information of entire nucleic acids - even after fragmenting - which improves assembly of the sequence after sequence determination. This means that if reads from different subpools align to the same gene they must originate from different transcript variants of this gene. Therefore sequence variation such as RNA editing or concentration differences between such transcript variants can be detected. Furthermore such differences can be compared between different samples. Of particular relevance are such comparisons between phenotypically different samples, to investigate the underlying causalities for this phenotype. "Full length" or "complete" in this context reads on the complete nucleic acids that are to be sequenced, e.g. as obtained after reverse transcription. It may comprise sequences of RNA starting from the 5' cap end up to the, but in most cases excluding the poly A tail, but may also relate to nucleic acids that are incompletely (reverse) transcribed, however without being artificially cut, e.g. by using endonucleases.

It is within the scope of this invention that the RNA was degraded or fragmented or digested by nuclease activity and the cDNA molecules derived from such RNA is only a partial sequence. Also the cDNA can be a partial copy of the RNA, e.g. oligo dT primed reverse transcription of mRNA is stopped before a full length cDNA copy is polymerized. This can be achieved through e.g. time restriction or through conditions where the reverse transcriptase stops polymerization at regions of secondary structure. Such a fragment can then be segregated by a common feature, e.g. the sequence preceding the poly A tail of an mRNA.

It is preferred that the pool of cDNA (cDNA libraries) contains nucleotides of the transcription start and/or end site, e.g. the first 25 and/or last 25 nucleotides. The pool of cDNA may also only consist of such first and/or end nucleotides. For example in CAGE (Shiraki-2003) 20 nucleotide tags are created that represent the 5' end of mRNAs. Of course such an approach will preclude the assembly of full length transcripts or the determination of their concentration. However such tags can be used to determine expression on a whole gene level, meaning the concentration of all transcription start sites can be measured. As only a short portion of an RNA will be sequenced sequencing depth increases and low level expressed genes will be more likely represented in the reads. However highly abundant transcripts will still be more often sequenced than low abundant transcripts. Therefore a segregation approach will increase the likelihood of low abundant start sites to be detected. For instance the short 5'tag sequences that are used to prepare a CAGE library can be segregated into fields of a matrix according to nucleotides on the 5' and/or 3' ends of such tag sequences. Therefore 5' tag sequences of low abundant transcripts will be more likely represented in a CAGE library that was prepared including a segregation step. Segregation can thus be performed on RNA, a cDNA thereof or other nucleic acids, e.g. RNA fragments, cDNA fragments or amplified nucleic acids therefrom.

The segregation step can be optionally repeated to obtain a different subpool with a different characterizing nucleic acid feature. This generation of further subpools can be performed sequentially or parallel to the generation of the first or other subpools.

The present invention in essence is a combination of selecting a pool of diverse RNA molecules, optionally generating cDNA, segregating the RNA or the cDNA, or any other nucleic acid derived therefrom, e.g. after amplification, optionally repeating the segregation for different parameters and fragmenting these segregated nucleic acids obtaining a pool of fragments. A fragment is considered a nucleic acid portion of shorter length than the complete nucleic acid molecule from which it is derived. Such fragments can be e.g. forwarded to Next Generation Sequencing approaches or other nucleic acid characterization methods. NGS is currently the foremost complete analyzing method. However, the present invention is neither limited nor dependent on NGS. Other sequencing technologies can similarly benefit from the inventive segregation method.

Often not solely the complete sequencing of the nucleic acids is required to clearly characterize a certain sub-pool distribution. Any other methods like specific interaction with molecular probes or melting behavior can be applied to describe the original nucleic acid pool through a unique signature. For instance molecular probes can be hybridization probes such as oligonucleotides that can hybridize to complementary sequences. Such principle is used in microarray analysis to investigate the expression of a large number of genes simultaneously. The most detailed analysis of gene (DNA) expression possible with such cDNA or oligonucleotide microarrays are exome or splicosome analysis. However also in these high resolution analysis the assignment of a signal to a particular transcriptvariant of a gene is not possible. However, as taught in the inventive method, when mRNA molecules or their full length cDNA copies are segregated into different subpools each subpool can be analyzed separately with a microarray. If two or more different subpools give a signal involving the same probe (spot on the array) the signal must belong to at least two different transcripts. This is of particular relevance when comparing the expression of different samples. Some differences in expression that cannot be distinguished without segregation prior to analysis can be detected if segregated. For instance a probe selective for a splice junction of a gene yields a relative signal of 100 in a first sample and 100 in a second sample. Therefore the expression ratio is 1 and not difference would be attributed. After segregating each sample into, e.g. 12, subpools and analyzing each of the subpools with a microarray two subpools are found in sample one the first with a relative signal of 90 and the second with a signal of 10. In the second sample the first subpool has a value of 10 and the second subpool gives a value of 90. Though the ratio of the combined subpools between the two samples is still 1, the ratio between the samples for the first subpool is 9 and the ratio for the second subpool 1/9. Therefore with segregation a difference in the expression of two transcriptvariants of a gene became detectable that could not be detected without segregation. In other words, if the signal originated from two different transcriptvariants then without segregation the one variant masked the signal of the second variant. When segregated each could be measured individually.
The same principle applies to next generation sequencing experiments. If reads in two subpools align to the same gene, it means that the reads must originate from different transcripts if the segregation power is 100%.
Furthermore segregating the transcriptome in terms of segregating transcripts from different genes as well as transcripts from the same gene into defined subpools is also a powerfull tool for the assembly of rather short sequence reads to longer or even full length sequences. In continuation the invention improves the alignment of large numbers of individual sequencing reads to determine the sequence of nucleic acids and/or their copy number.

In one embodiment the generation of fragment (partial) sequences is done during the sequencing step, rather than first fragmenting and then sequencing such fragments. Here a random (universal) primer is used to prime the sequencing reaction within a single molecule. Therefore the sequencing reaction will in most cases create a fragment sequence from within the molecule. If the molecule had a subpool specific label this label could be read out after the sequencing reaction, providing the fragment sequence with the subpool specific label. The same molecule could be subjected to further sequencing, thus providing a multitude of fragment sequences that can be assembled to a contig or a full length sequence of the nucleic acid molecule, the RNA or transcribed cDNA. As a specific nucleic acid can be present in multiple copies, such sequencing could be done also in parallel. Here a multitude of random (or universal) primers prime the sequencing reaction of a multitude of nucleic acid molecules producing a multitude of fragment sequences that as a whole can be used to align or assemble the sequence of the segregated nucleic acids.

It is within the scope of this invention that fragments are ligated to each other prior to sequencing.

Nucleic acids are linear polymers of single nucleotides. These molecules carry genetic information (see triplet code) or form structures which fulfill other functions in the cell (e.g. regulation). The nucleic acids which are analyzed by the present invention are ribonucleic acid (RNA). RNA (sequencing) analytics is a particular difficult task due to the complexity of RNA populations in single cells. The invention relates to the identification (particular sequence determination) of all types of RNA in a cell, including mRNA (transcripts), microRNA, ribosomal RNA, siRNA, snoRNA.

The transcriptome is the set of all RNA molecules, or "transcripts", produced in cells. Unlike the genome, which is roughly fixed for a given cell line, the transcriptome varies with the kind of cell, tissue, organ and the stage of development. It can alter with external environmental conditions. Because it includes all transcripts in the cell, the transcriptome reflects the genes that are being actively expressed at any given time, and it includes degradation phenomena such as transcriptional attenuation. Transcriptomics is the study of transcripts, also referred to as expression profiling. An inventive benefit in using the inventive Segregation method on RNA samples is that transcripts with low copy numbers or any other type of RNA which is present in the sample in a low concentration has an increased chance to be sequenced and analyzed in the subpool. One drawback of Next Generation Sequencing is that highly abundant nucleic acids reduce the chance that fragments of low concentration are sequenced. The inventive segregation allows the differentiation of high copy number entities with low copy nucleic acids. Thus preventing that such low copy nucleic acids are excluded from detection - or in any other preceding step such as e.g. during amplification.

The general principle is to reduce the complexity of a pool of nucleic acids by sequencing smaller segregated portions. These smaller portions are called sub-pools. In a preferred embodiment all sub-pools together contain all nucleic acids to be analyzed of the original pool. However, it is in principle not necessary to analyze all RNA molecules and thus some sub-pools can be ignored or are not even created/may remain empty. There are three main factors that contribute to the complexity of nucleic acids pools.

The first factor is determined by the combined length of the individual different sequences. Because the sequence is encoded through 4 bases (T and U are considered equivalent for carrying the same information) the complexity increases as a variation, equal to four to the power of length. However genomes contain redundant information like repeats or any other kind of order, e.g. that arises through the evolution of genes. Therefore different genes can contain stretches of the same or very similar sequences. This creates ambiguity in the de novo assembly of contigs or full length transcript sequences and limits the length of contigs that can be built. Even in alignment processes where a reference sequence is available such ambiguity restricts the alignment of individual reads. This ambiguity increases with decreasing read length of the sequencing process. In transcriptome analysis this ambiguity is even higher as one gene (or genomic region) can code for more than one transcript. Different transcripts from the same gene (sometimes referred to as transcriptvariants) such as splice variants are very similar in terms of sequence composition. Therefore most reads arising from transcriptvariants cannot uniquely be assigned. E.g. even if a splice junction is detected, it is not known if such a junction belongs to one or more transcripts.

The second factor is determined by the number of different sequences within a sample. The complexity increases with the number of permutations, therefore with the factorial of different sequences. Two sequences have two possibilities to arrange, three sequences have six possibilities and so forth.

The third factor is the difference in copy numbers (transcript concentrations) and to lesser degree the amount of precognition about these differences, e.g. if it is known that the difference of certain copies is in the order of 1/1.000. Each different sequence belongs to a group which is characterized of having one particular copy number. The level of distribution of these groups determines the complexity which is introduced through concentration differences.

The inventive segregation can help to distinguish different RNA molecules of the original sample pool. This segregation step can also be repeated once or more. Repetition herein shall not be interpreted that additional segregation steps have to be performed after the first segregation step - which is of course one option - but also relates to performing one or more segregation steps simultaneously. Thus, one or more subpools are generated and in each subpool specific nucleic acids are present (or enriched) which share a common feature and all other nucleic acids without that shared distinctive nucleic acid feature can be excluded from each pool (or at least are not enriched).

These factors contribute directly to the difficulty of determining the correct sequence and concentration of all and in particular rare molecules within a sample. The general principal of the present invention is the constituting of sub-pools where these factors can be controlled, and simultaneously the complexity of the pool reduced, before sequencing reads are generated. Thus, the method simplifies the in-line sequence alignment. Subpools emerge through segregation methods which are within the scope of this invention.

In a preferred embodiment of the present invention the method further comprises determining the sequence or a partial sequence of the fragments of the first subpool and optionally further subpools. This sequence of the fragments or a portion thereof can be determined by any suitable method known in the art. Preferred are sequence determination methods that can be scaled to high throughput sequencing methods, in particular Next Generation Sequencing. In such a method sequence length of at least 5, preferably at least 8, at least, 10, at least 15, at least 18, at least 20, at least 22 nucleotides of the fragments or more can be determined. Preferably the full length sequence of the fragments are determined. If only portions of the fragments are sequenced this can be either portions of the 5' or the 3' end or internal portions which can be selected for with specific or unspecific (e.g. random) primers.

Determining a partial sequence of a nucleic acid preferably comprises determining a sequence portion of at least 10, preferably at least 15, at least 18, in particular preferred at least 20, even more preferred at least 25, nucleotides but excludes determining the complete sequence of the nucleic acid. According to the present invention it is possible to either generate fragments of the segregated nucleic acid molecules by random fragmenting or obtaining fragment copies (e.g. amplifying portions of the nucleic acid molecules) and subsequently determine the sequences thereof or to determine a sequence or partial sequence of a fragment of said segregated nucleic acid molecule and, preferably align at least 2, preferably at least 3, in particular preferred at least 4, at least 6 or at least 8 sequences or partial sequences to a joined sequence. According to this option, it is not necessary to physically provide such fragments but possible to only obtain sequence portions which can be determined from the nucleic acid molecules themselves without a physical fragmenting step and create a joined sequence by aligning such partial sequences. According to this embodiment, it is thus not necessary to provide specific labels providing the information of the segregated pool since the sequences are determined directly on the nucleic acid molecules of the sub-pool. This is possible by e.g. random priming, a primer extension from inside the nucleic acid molecule, or by e.g. nano-pores which can read out at any point of the sequence, therefore creating "fragment reads". Such reads can then be aligned as described herein. Nano-pores are used according to this option of the invention.

In particular, it is not always necessary to provide full-length sequences of all fragments provided. It is also possible to determine missing sequence portions from other fragments which may e.g. overlap and thus provide the same sequence as is lacking in the incompletely sequenced fragment. E.g. it is usually more efficient only to determine the sequence from one end of the fragment and to sequence a partial sequence as mentioned above of e.g. at least 10 nucleotides. Such partial sequences can then be aligned to a joined sequence. Although according to one embodiment it is possible to determine the full-length sequence of the segregated nucleic acid molecule by the method of the present invention, it is also possible to only determine portions thereof long enough to identify said nucleic acid molecule.

Preferably the information about the sub-pool origin escorts the nucleic acid molecule and each of its fragments during the sequencing run. On one hand the sub-pool information can be passed on through labeling. Every fragment may receive an identifying nucleotide sequence (e.g. adding a subpool specific sequence tag of e.g. 1, 2, 3, 4, 5, 6, 7, 8 or more subpool related nucleotides), reporter module like a fluorescent dye, nano-dots, or others. It is preferred that the subpool specific label is a nucleotide sequence (barcode) that is added to the fragment. Furthermore it is preferred that the barcode is read out after or during sequencing the nucleic acid fragment. On the other hand, the sub-pool information can be perpetuated through spatial or temporal separation, which means that each sub-pool is sequenced in a different area (cluster on a slide) in the machine or discriminating time slot, e.g. each subpool may be sequenced sequentially. No additional process conduct is needed for most of those procedures. In the case of individual labeling with a reporter molecule, the reporter signal has to be identified and connected to the read.

The individual sub-pools can be sequenced separately. Reads of each sub-pool are aligned either to the genomic blue print, or they are aligned de novo by comparing them with all other reads within the same sub-pool and not the total pool. Therefore, the,complexity of the original sample pool is greatly reduced.

Abundant RNA molecules, in particular transcripts, interfere only in one sub-pool of their appearance and so compromise its reading depth, but not the rest of the subpools. Because the probability of reading individual fragments is proportional to their relative concentration within the pool or sub-pool respectively, fragments which are present in just a thousandth will on average only be read once while having read the other fragment(s) thousand times.

For alignment of the reads, all reads are grouped, and where possible oriented according to their sub-pool address. Second, all reads are aligned to each other or to the blue-print sequence data base. The alignment must fulfill all boundary conditions, if e.g. further information of the complete sequence such as length is known in addition to the sub-pool information.

However, often it is not necessary to completely sequence a fragment but only obtain a portion of its sequence. Sometimes such a portion is sufficient to identify the nucleotide or align other sequenced portion of other fragments to a full sequence (e.g. if the fragments contain overlapping sequences).

Apart from sequencing portions of the fragments it is also possible to only obtain fragments i.e. smaller nucleic acid molecules with portions of the original nucleic acid, and determine the sequence or a portion therefrom. "Generating fragments of said segmented nucleic acid molecules" thus, also relates to obtaining a fragment which contains any kind of sequence portion within the limitations of the claims. Fragmenting can be by e.g. physical means be either in a sequence dependent way, e.g. by endonuclease digestion or by sequence independent means such as by a physical means like sonication or shearing. Generating the fragments further relates to obtaining fragment copies. The nucleic acid molecule can be e.g. amplified to further copies which are in turn fragmented. If a random fragmenting process is used this can result in generation of different fragments for each nucleic acid molecule. On the other hand, if a sequence dependent method is used, e.g. restriction endonuclease digestion or sequence specific amplification all fragments of one nucleic acid molecule will be the same. Furthermore, it is possible to generate fragments by amplification, i.e. sequencing fragments. This can e.g. also be done in a sequence independent or dependent method, in particular preferable by random priming in order to obtain internal sequence portions with said fragments. Example sizes of the fragments or determined partial sequences are e.g. at least 10, at least 20, at least 25, at least 30, at least 35, at least 40 nucleotides. Fragments or determined partial sequences can be up to 20,000, up to 10,000, up to 5,000, up to 4,000, up to 3,000, up to 2,000, up to 1,000, up to 800, up to 700, up to 600, up to 500, or up to 400 nucleotides long. The preferred ranges are of 10 to 10,000 nucleotides, preferably of 25 to 500 nucleotides.

It is within the scope of this invention that fragments are joined prior to sequencing. It is preferred that such joined fragments are interspersed by different sequence stretches that allow sequencing primers to prime consecutive rounds of sequencing.

The segregated nucleic acid molecule or the nucleic acid molecule to be segregated can be either single stranded or double stranded. In cases where single stranded molecules are segregated the strandedness of a fragment in relation to its parent molecule is clear as it has a 5' and a 3' end. When double stranded nucleic acid molecules are used then there needs to be a distinguishing property on one strand but not the other (e.g. methylation) as the double strand has a 5' and 3' end on both strands. In cases where a feature (preferably a sequence portion) at the 5' and/or 3' end of the RNA or cDNA was used as the nucleic acid feature the orientation of the molecule is still known before fragmentation. Therefore one of the two strands can be used for fragmentation. One of the two strands can be selected for by any means known to the art. E.g. the end of one strand can be labeled during the segregation. For instance one of the PCR primers may contain a labeling group such as biotin and be selected for afterwards using column chromatography with an avidin coupled matrix. Another possibility is to use one primer that has a 5' phosphate and another primer that has no 5' phosphate and subject the PCR products to a lambda exonuclease that preferentially will digest the strand that has a 5' phosphate. By preserving the strandedness or the strand information of the nucleic acid molecule throughout the segregation and fragmentation, the performance of subsequent assembly or alignment is improved. For instance if the strandedness of the fragments is preserved, each fragment can be aligned to the plus or minus strand of the genome, thereby distinguishing between sense and antisense transcripts. The same holds true for cluster building or the de novo assembly of transcripts as again sense and antisense clusters/transcripts can be distinguished. It is therefore preferred that during fragmentation the strandednes's or strand information is preserved, preferably by selecting for one strand, e.g. through a lambda nuclease digest of the other strand. It is possible during segregation to select one strand to be segregated (either the sense or anti-sense strand) or to label the selected strand in order to maintain strand information. Preferably the fragments of the selected strand are labeled according to the strand information and possibly also for pooling information (e.g. bar-coding as mentioned above).

In further preferred embodiments at least 2, 4, 5, 6, 7, 8, 9, 10, 12, 14, 16, 18, or at least 20 nucleotides, in particular consecutive nucleotides of these fragments are sequenced.

The original pool of potentially diverse RNA molecules can be of any source, in particular of any biological sample, preferably of a virus, prokaryote or eukaryote. The inventive complexity reduction method is important for any sorts of RNA sequencing approach, even when using a single cell which contains a diverse transcriptome but of course also samples which contain more than one cell, in particular samples of diverse origin, e.g. containing many different cells of diverse organisms or similar cells with different or modified gene expression (e.g. tumor cells).

In a particular preferred embodiment of the present invention the nucleic acid feature used for segregation is a given nucleotide type, preferably selected from any one of A, T, U, G, C, at a certain position in the nucleic acid molecule, preferably the position being within 100 nucleotides from either the 5' or 3' terminus or both of the nucleic acid molecule. Such methods, that select for one or more specific nucleotides, e.g. to obtain full length sequence source disclosed in the WO 2007/062445 (incorporated herein by reference). In a preferred embodiment the inventive segregation step may thus comprise segregating nucleic acids from said template RNA or cDNA pool, selecting for potentially different templates with at least one given nucleotide type at a certain position being within 100 nucleotides from either the 5' or 3' terminus of the full length template nucleic acid molecule sequence shared by the segregated templates, thereby providing at least a first subpool of nucleic acids.

According to the present invention it is possible to amplify or select for specific nucleic acid molecules in a segregation step by using, e.g. a primer, which is specific for e.g. one end (either the 3' or 5' end) of the RNA or cDNA and containing one or more further nucleotides specificities which act to segregate the nucleic acid molecules according to the complementary nucleotides after the (universal or wobble) primer portion. If full length RNA should be segregated then it is possible to use primer specific for the ends, e.g. the polyA-tail (or polyT-tail on a cDNA corresponding thereto) or to attaching artificial tails onto the RNA or cDNA and using primers specific for this tail. The primers can be specific for the next 1 to 100, preferably 1 to 10 nucleotides, e.g. the next 1, 2, 3, 4, 5, 6, 7, 8, 9 or 10 nucleotides. By using wobble nucleotides on said primers it is also possible to select for specific nucleotides after these ends. Preferably the specific distinguishing nucleotides are within the first 100 nucleotides from either the 5' or 3' terminus of the nucleic acid molecule. It is of course also possible to use primers to select any internal region wherein the nucleic acid molecules can be separated in the segregation step.

The same principle mentioned above for primers of course also applies for oligonucleotide probes which can be specific for such a distinguishing nucleotide type.

Preferably, the nucleic acid molecules are selected for common nucleotides within the 10 nucleotides next to the 5' and/or 3' terminus, preferably for one or more common 5' and/or 3' terminal nucleotide types.

These primers or probes preferably are used in combination with primers or probes which are selected for a different nucleic acid feature. Such primers can e.g. be used separately or sequentially to generate subpools specific for the nucleic acid feature. Such primers or oligonucleotides used in a combination (i.e. "primer matrix") can e.g. be primers which have a universal part and a distinguishing part wherein the distinguishing part is e.g. A in the first primer, T in the second primer, G in the third primer and C in the fourth primer. Preferably, more than one nucleotide is used as the nucleic acid feature and the combination can e.g. be primers or oligonucleotide probes ending with AA, AT, AG, AC, TA, TT, TG, TC, GA, GT, GG, GC, CA, CT, CG, or CC, thus separating nucleic acids with complementary nucleotides into different sub-pools. In a further preferred embodiment the nucleic acid feature contains 3 or more, e.g. 4, 5, 6, 7, 8, or more specific nucleotide types. In a further preferred embodiment, combinations of primers are oligonucleotides selecting for distinguishing nucleotides at both the 5' and/or 3' terminus, e.g. both primers or probes being specific for the two or more 5' nucleotides and the two or more 3' nucleotides.

As mentioned above it is also possible to select for internal regions wherein it is also possible to use a combination of such a primer pair which selects for two nucleotide types on each side of the amplicon. Internal regions can alternatively also be selected for by using end-specific primers or probes having a certain number of unspecific nucleotides (e.g. wobble or universal nucleotides) prior to the complementary nucleotides for the specific internal region.

In a preferred embodiment the nucleic acid feature that is used for segregation, is used during the assembly (or alignment) of short reads as a qualifying property of the assembled (or aligned) sequence. For instance if the nucleic acid feature was a certain length or length range then the qualifier for a correctly assembled sequence would be such length or length range. If the nucleic acid feature was a certain sequence then when sequencing fragments of this nucleic acid, that are e.g. 36 bases long, then in addition to the 36 bases another n bases are known for each fragment, where n is the number of bases of the nucleic acid feature. If for instance the nucleic acid feature was 6 known bases on the 5' side and 6 bases on the 3' side of the molecule then in addition to the 36 bases of each fragment 2x6 bases are known to be within a certain distance (the length of the fragmented molecule) from the sequenced fragment. Therefore if the nucleic acid feature was a certain sequence then this sequence must be again contained within the assembled sequence. It is preferred that the nucleic acid feature is at a certain position of the segregated nucleic acids, preferably at a certain distance from the 5' or 3' end of the template RNA or cDNA. Preferably the nucleic acid feature is a sequence and the sequence is used during assembly. The nucleic acid feature may comprise two sequence portions, e.g. of 2, 3, 4, 5, 6, 7, 8, 9, or 10 known nucleotides, positioned in a certain base distance, e.g. in a distance of e.g. 20 to 10000 nts, preferably 30 to 5000 nts, in particular preferred 50 to 1000 nts.

In a preferred embodiment the segregated nucleic acids contain the full length sequence of the template RNA or cDNA. This will greatly increase the de novo assembly of contigs or even full length sequences as all fragment reads generated during a sequencing process can be aligned within a subpool, i.e. with the fragment or partial sequences obtained from one subpool.

If the nucleotides of the 5' and/or 3' end of the template full length RNA were used as the nucleic acid feature(s)for segregation, the nucleotides of the start and/or end site of the full length RNA molecule are known for all fragments of such subpool. Such information allows e.g. to position fragments or their assembled contigs correctly on the plus or minus strand of the genomic DNA, thus separating sense and antisense transcripts of a gene. In a preferred embodiment the RNA molecule used according to the inventive method is a full length RNA. Full length RNA can e.g. be selected with the above mentioned method. The same also applies to full length cDNA corresponding to the full length RNA. As used herein the term "full length RNA" or "full length cDNA" is defined as RNA or DNA that includes a sequence complementary to the RNA sequence from the first base to the last base of the RNA. Such a method is e.g. disclosed in WO 2007/062445 (incorporated by reference) and comprises amplification selective for end specific nucleic acid features e.g. by performing a segregated amplification or selection (as described herein) on full length RNA. In case of RNA molecules that have a cap and/or a tail (polyA-tail) as is the case for most eukaryotic mRNA, "full length RNA" is defined as RNA that includes a sequence complementary to the RNA sequence for the first base after the cap e.g. the RNA 7-methylguanusin cap to the last base before the tail, polyA-tail, of the RNA template.

In order to bind primers during amplification and/or sequencing reactions to ends of nucleic acids or the fragments it is possible to attach linkers or adaptors to the nucleic acid molecules or fragments to allow primer binding.

By ordering a pool of RNA molecules into the inventive subpools it is possible to highly decrease complexity of the original sample, generating subpools with fewer nucleic acid entities and therefore increase the chance of detecting nucleic acids or successful sequencing and assembling afterwards.

In preferred embodiments the nucleic acids are divided into subpools wherein at least 10% of all subpools comprise the average amount of nucleic acids of all subpools +/- 50%. By employing a suitable segregation method for the given sample to divide the nucleic acids evenly into the subpools the complexity reduction method is sufficiently used. Of course, further subpools may exist wherein fewer nucleic acids are present, e.g. even empty subpools without any nucleic acids of the original pool which can be used as control reference. In preferred embodiments at least 15%, at least 20-, at least 25%, at least 30%, at least 35%, at least 40% of all subpools comprise the average amount of nucleic acids of all subpools +/- 50%. This error margin of +/-50% is in preferred embodiment up to +/- 50%, up to +/- 45%, up to +/- 40%, up to +/- 35%, up to +/- 30%, up to +/- 25%, up to +/- 20%.

Preferably the sample comprises at least one, preferably two, 3, 4, 5, 6, 7 or 8 rare RNA molecules. Rare can mean a concentration of below 1%, below 0.5%, below 0.1%, below 0.05%, below 0.01% (100ppm), preferably below 50ppm, below 10ppm, below 5ppm, below 1ppm, below 500ppb, below 100ppb or below 50ppb. Preferably at least 1, preferably 2, at least 4, at least 6 or at least 8 rare nucleic acids are in the sample to be analyzed.

In a further embodiment the nucleic acids are divided in subpools wherein at least 10% of subpools contain 2 or less nucleic acids, preferably 1 nucleic acid. Such a high dilution is in particular favorable for very rare nucleic acids that would be hard to detect if further nucleic acids would be present from the original pool, in particular in the original concentration.

In a further preferred embodiment the step of segregating the nucleic acids comprises specifically amplifying the nucleic acids from said template pool. In particular, the amplification is performed by nucleotide extension from a primer, preferably by PCR, in particular preferred wherein the amplification is performed by nucleotide extension from a primer, preferably by PCR, in particular preferred wherein the amplification is performed by using primers which select for at least one, preferably at least two, in particular at least two adjacent, different nucleotides after an unspecific primer portion whereby nucleic acid molecules are amplified which comprise the selected nucleotide as the nucleic acid feature specific for a subpool.

The above mentioned fragmentation step of the inventive method may be the first step used for sequence determination steps. Determining the sequence of the nucleic acids of the subpool may comprise, fragmenting the nucleotide molecules of the subpool as mentioned above, attaching a subpool specific label to each fragment of a given subpool, determining nucleotide sequences of fragmented polynucleotides of combined pools (or alternatively determining nucleotide sequences of separate pools with or without attaching a label), assigning fragment sequences to a nucleotide molecule depending on a subpool-specific label and overlapping sequences with other fragments, thereby determining the sequence of the nucleic acids.

Thus, in preferred embodiment subpool-specific labels are attached to the fragments. The subpool-specific labels can be nucleotides, which are preferably co-determined during sequence determination.

In further preferred embodiments the nucleic acids of the original pool are divided into at least 2, preferably at least 3, at least 4, at least 5, at least 6, at least 7, at least 8 subpools during the segregation step, which nucleotides each share a different nucleotide characteristic for each subpool.

In preferred embodiments primers or probes used for selecting nucleic acids in the segregation step are preferably immobilized on a solid surface, in particular a microarray or chip. The same type of segregation as described above for the distinguishing the nucleic acids can also be performed for distinguishing different fragments during the sequencing step.

In a particular preferred embodiment the inventive method further comprises amplifying the nucleic acid molecules, preferably after segregation, prior to determining the sequence, in particular preferred wherein said amplification is by PCR and at least one nucletide molecule is amplified to the saturation phase of the PCR. In particular preferred at least 10% of the different nucleotide molecules are amplified to the saturation phase of the PCR. Such an amplification reaction can be used to normalise the concentration of nucleic acid molecules in the pool or sub-pool. A PCR reaction e.g. has an exponential phase in which the nucleic acid molecules are essentially doubled in each PCR cycle. After the nucleic acid molecules reach a certain concentration in relation to the primer concentration, competitive reactions start to inhibit the amplification. Thus, amplification of abundant nucleic acid molecules starts to slow down due to the self inhibition of the nucleic acid molecules which can prevent primer binding. Alternatively reaction components such as primers, dNTPs are used up. This phase is called the saturation phase.

Preferably, highly abundant nucleic acid molecules reach this saturation phase and are inhibited from amplification whereas low abundant molecules continue amplifying exponentially. Preferably at least 10%, in particular preferably at least 20% of the different nucleic acid molecules enter this saturation phase. These amplification reactions can e.g. be monitored by using qPCR (quantitative PCR). Of course, said reactions occur in normal PCR reactions (but may be unmonitored) or other amplification reactions with self inhibition, e.g. after 20, 22, 24, 26, 28, or 30 amplification cycle, which are preferred minimum cycle numbers for the inventive amplification.

When segregating subpools in parallel e.g. through amplification in a PCR, subpools containing highly abundant transcripts will reach the saturation phase earlier. Therefore transcripts in subpools that do not contain these highly abundant transcripts will still be amplified in later cycles, when the subpools that contain the abundant transcripts are already in saturation phase. Therefore when sequencing all these subpools rarer transcripts get a higher chance of being detected.

The inventive sub-pooling procedure can also be used to remove high copy transcripts, e.g. exclude sub-pools with high abundant nucleic acid molecules from sequence determination. Preferably, such sub-pools with high abundant nucleic acid molecules that are excluded from sequence determination are subpools comprising more than 100%, particularly preferred more than 150%, even more preferred than 200%, particularly preferred more than 300%, e.g. more than 400%, such as more than 500%, particular preferred more than 1000%, nucleic acid molecules above the average amount of all sub-pools which may contain all the nucleic acid molecules of the sample. Such sub-pools can e.g. be subpools which comprise nucleic acid molecules that constitute e.g. more than 0,1%, more than 0,5%, or even more than 1%, e.g. more than 2% or more than 5% or more than 10% of the entire original pool. Abundant transcripts to be excluded or normalized by this way are e.g. of housekeeping genes, GAPDH, actin, tubulin, RPL1, ribosomal proteins, or PGK1.

The present invention is further illustrated by the following figures and examples without being limited thereto.

### Figures:

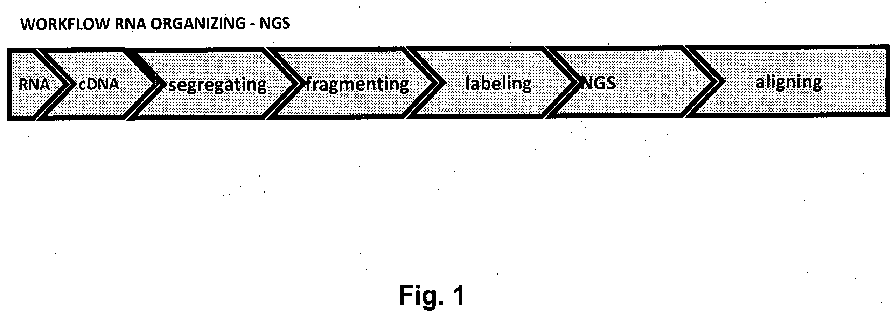

Fig. 1: Workflow of the Segregation-NGS method for RNA.
Fig. 2: Simulation of the number of genes as function of the mRNAs (total copy numbers of all gene transcripts) through a log-log-normal function. Active genes G, 16,657, total transcripts T, 3.8 Mio, most common transcript number, 10, scale value of the log-log-normal function µ, 1 and shape parameter δ, 0.4.
Fig. 3: Exponential decay function describing qualitatively the relationship of the number of transcripts vs. genes according to parameters tₛₜₐᵣₜ, 33, t_{end}, 1, the sum of all genes, 25,200 and a 4-fold amount of transcripts (100,269).
Fig. 4: Exponential decay function describing dependency of the mRNAs (copy number) vs. transcripts according to parmeters cstart, 10,000, c_{end}, 1, decay constant τ of 0.0522, the sum of all transcripts is 100,128 and the sum of all copy numbers is 3.8 Mio.

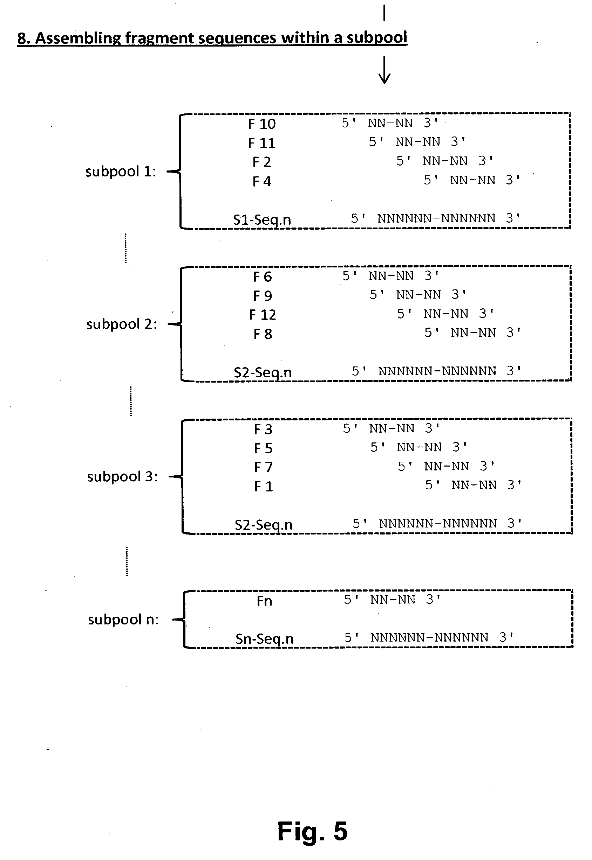

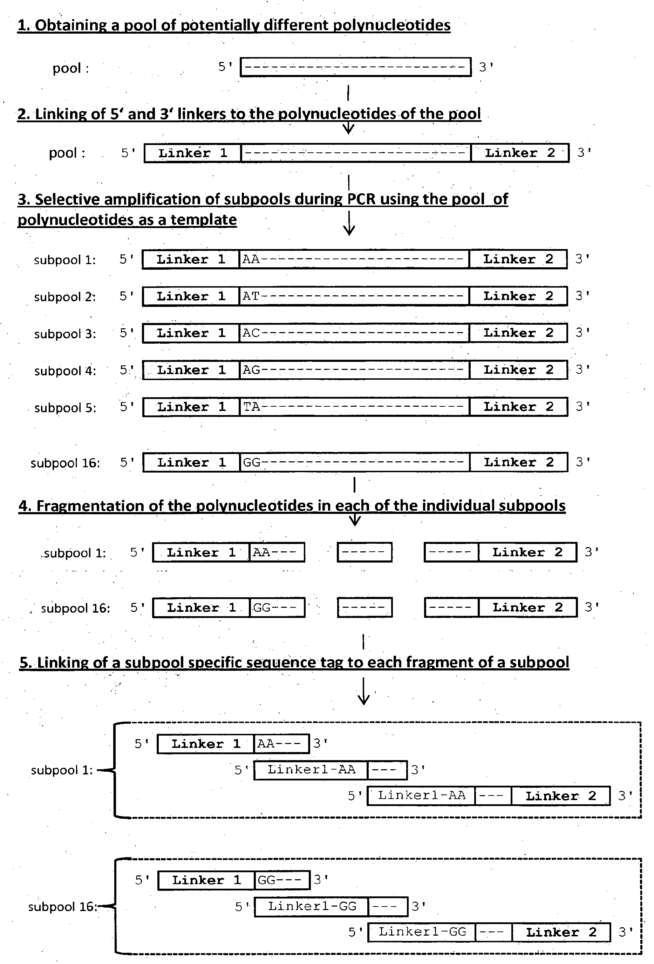

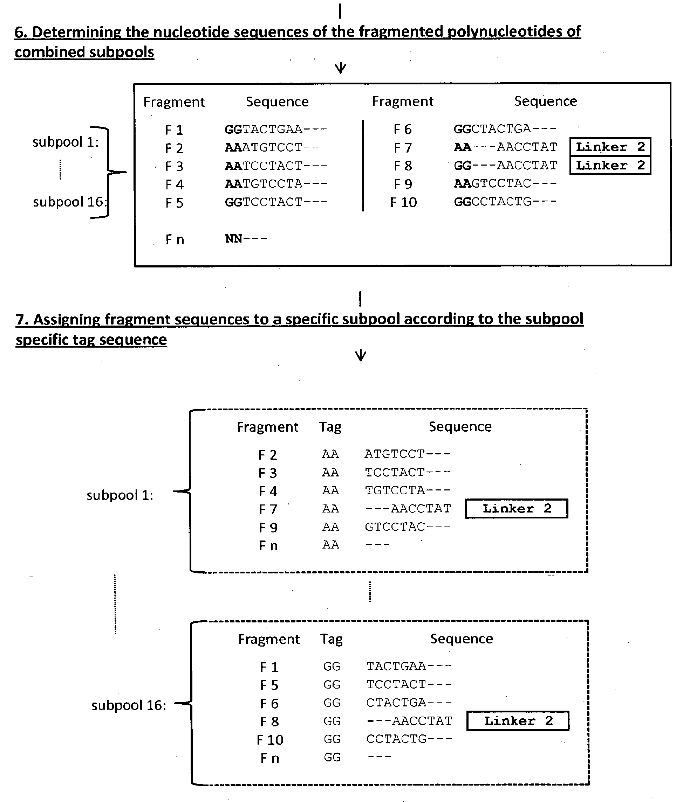

Fig. 5: General subpooling and fragmentation workflow.

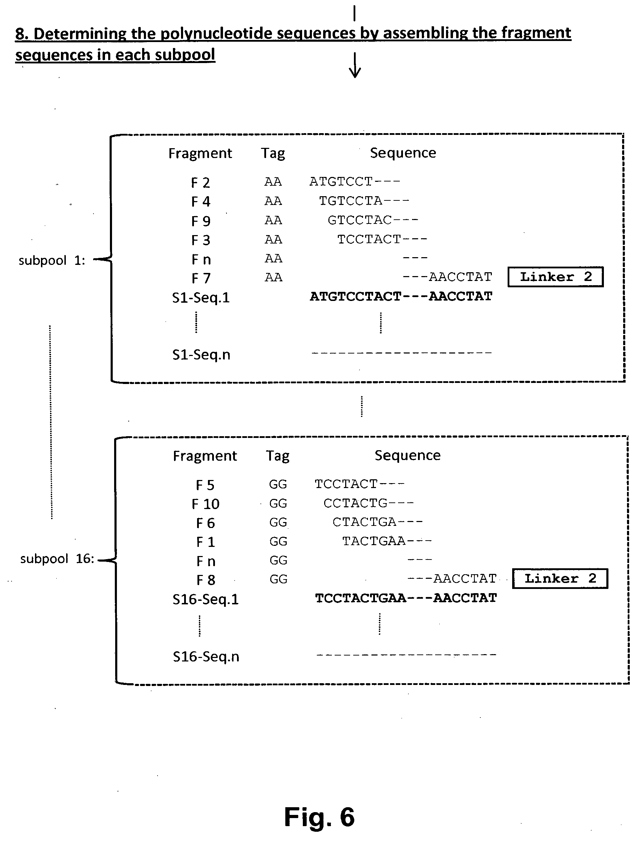

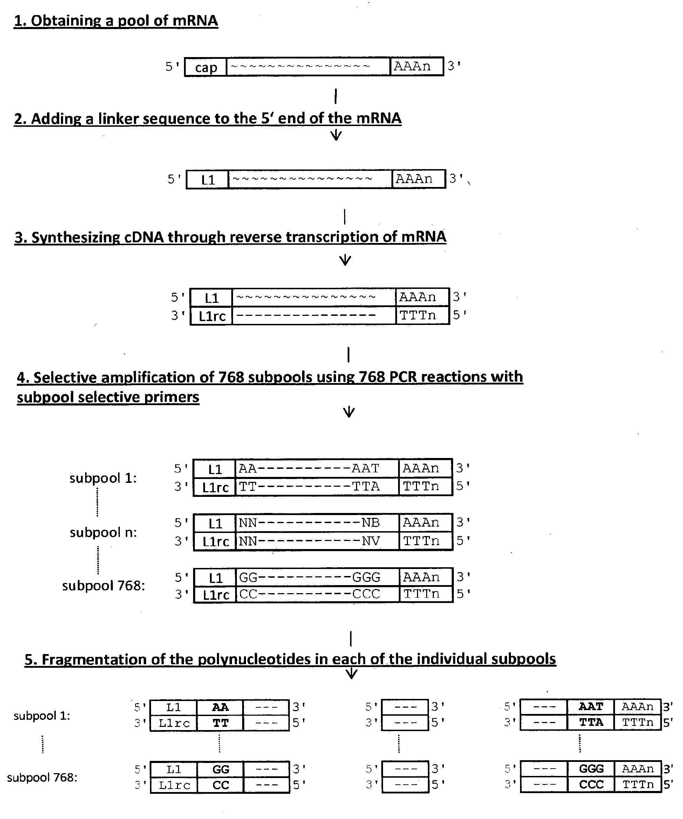

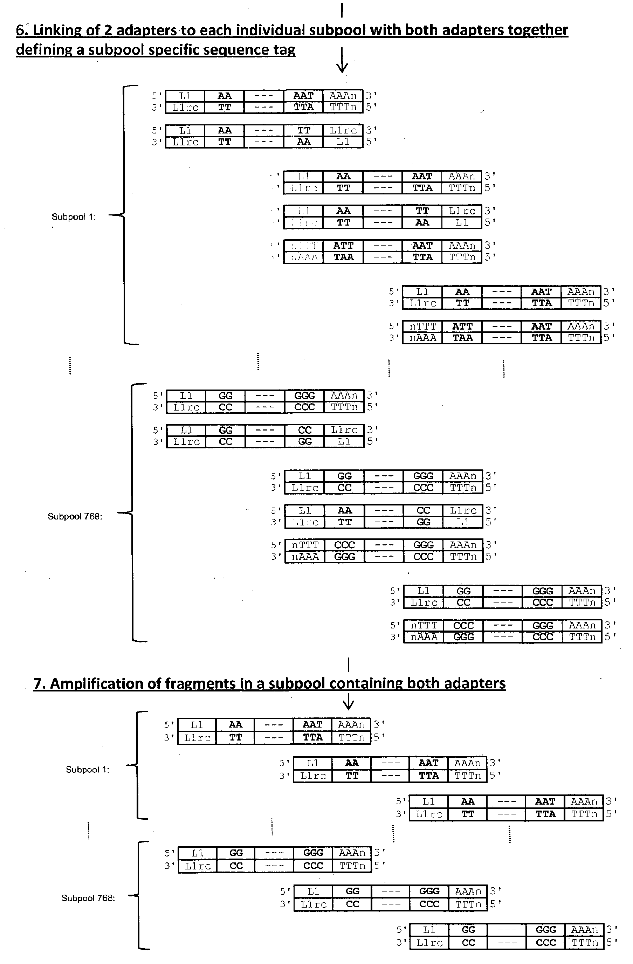

Fig. 6: General principle using nucleotide specific amplification (segregation). In this example the first two nucleotides at the 5' end used to define the subpools also become the sequence tag.

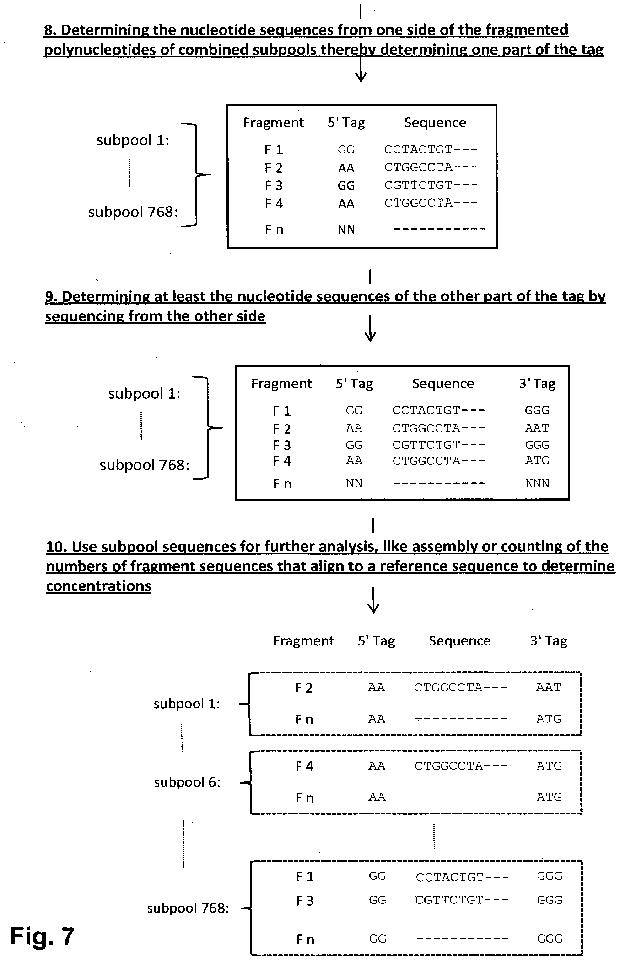

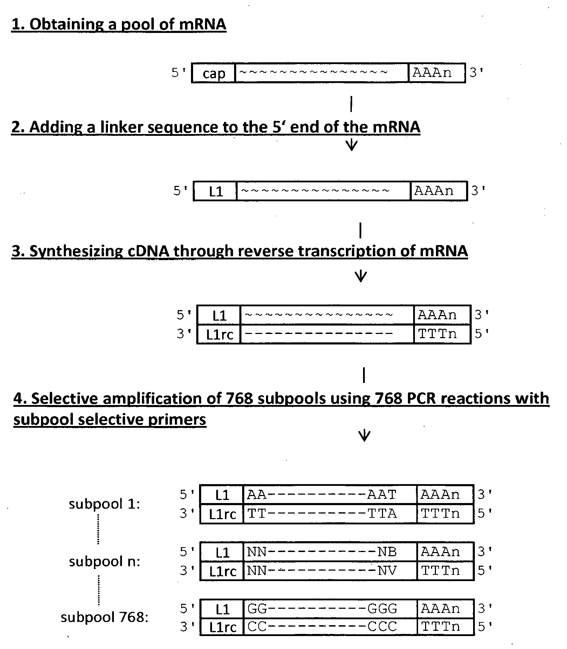

Fig. 7: RNA matrix segregation. In this example it is noteworthy that fragments F2 and F4 are sequence identical and could not be distinguished unless the segregation into the sub-pools was performed (see step 10). Adding a linker sequence to the 5' end of the mRNA as shown in step 2 can be done by any methods known in the art, such as Oligo capping (Maruyama 1994).

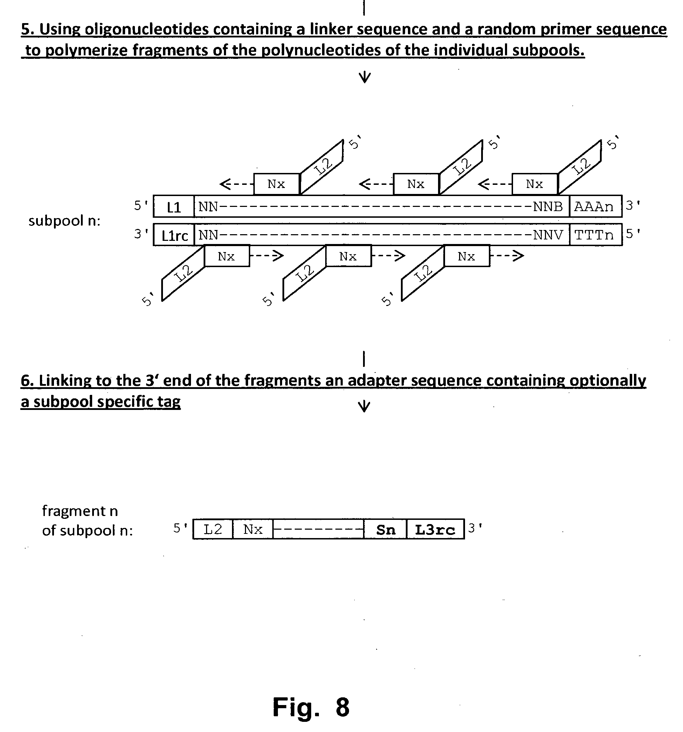

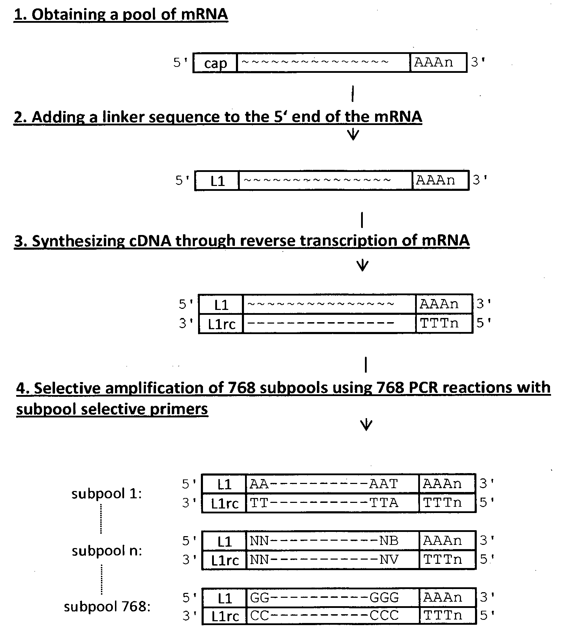

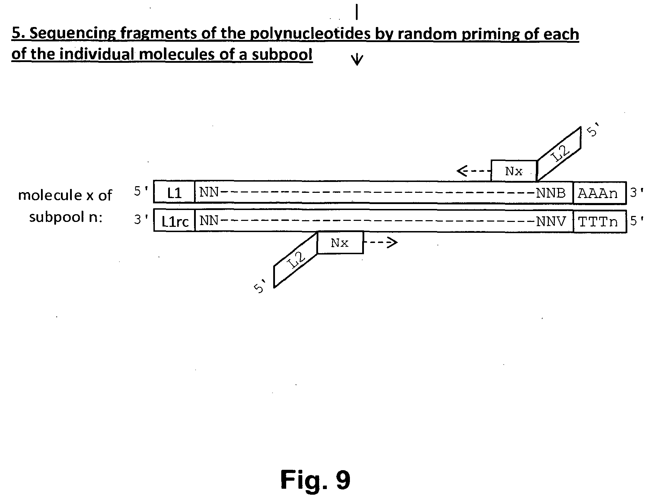

Fig. 8: Creating fragments by random primed polymerization Steps 1 to 4 are the same as in Fig 9. Shown is only subpool n. Sn in step 6 represents the subpool specific tag.
Fig. 9: Random primed sequencing, producing fragment reads. Steps 1 to 4 are the same as in Fig 7. In this example the molecule x of the subpool n is double stranded, each strand can serve as a template for sequencing. The random primer is bound to the surface of the sequencing chip. Single strands of each molecule of a subpool are hybridized to the primers on the chip. As the random primers can hybridize to any part of the molecule, the sequencing will produce "fragment" reads from the molecule.

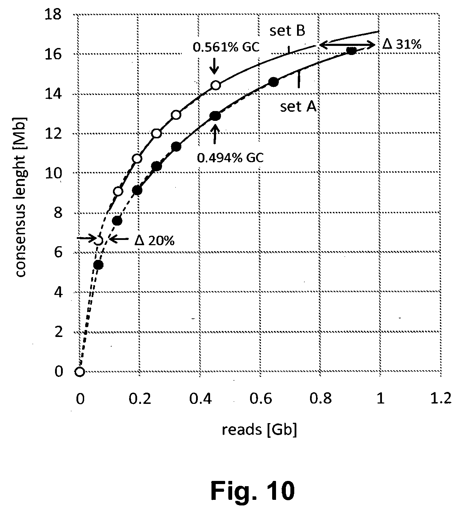

Fig. 10: Comparison of mouse genomic coverage which has been obtained by NGS read alignment from one non-segregated sample (set A) and one segregated sample (set B) of a 6 out of 12 subpool matrix (1x1). The consensus length (y-axis) describes the total length of uniquely detected sequences. On the x-axis the sum of reads in gigabases is depicted. The average read length was 65 nucleotides. The dashed line connects data points which have been obtained by randomized drawing read subclasses and aligning them separately to the mouse genome. The solid line is on inter- and extrapolation of the data points. GC, genomic coverage.

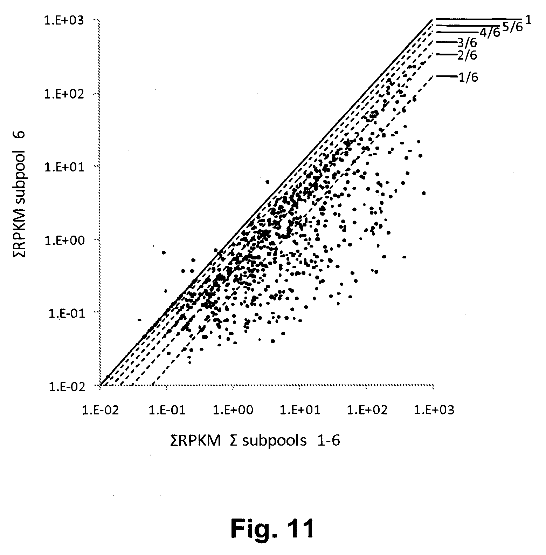

Fig. 11: Scatter-plot comparing expression of genes in one subpool (subpool 6) versus the 6 combined subpools from set B in example 1. Gene expression is depicted in snRPKM, that is RPKM (Mortazavi 2008) normalized to the sum of all reads in all 6 subpools. A randomized draw of 10% of all values was chosen to dilute the number of datapoints for better visualization. The diagonal lines in the double logarithmic scale depict the segments of the sixth parts. Shown in the graph is the central section with snRPKM values between 0.01 and 1000. The 6 values above the 6/6 line are caused by the ambiguity of the alignment algorithm used by CLC software.
Fig. 12: The subpool distribution of the 15 most abundant genes of set B in example 1 is shown. Genes are represented in different concentrations in different subpools, showing that transcript variants of different genes are segregated representing different transcript variant concentrations.
Fig. 13: Transcription start site analysis of gene Nmnt with start sites assigned by reads of RNA-seq, 0 and 1x1 matrix experiments. The genome annotation is schematically drawn and shows the start region of Nnmt. Individual reads are depicted with their respective position. The relative frequency of base reads corresponds to the dark grey area in the line "frequency of the read sequences".

### Examples

**Example 1:** cDNA segregation by end-specific matrix separation followed by NGS analysis. For oligonucleotides used see table 1.

2 µg of purified total RNA of a mouse (C57Bl/6) liver sample was primed by an oligo that contains a V (being either C,G or A) anchored oligo-dT sequence (Seq-2; Linker2-T₂₇-V) at its 3' end and was reverse transcribed to generate cDNA. Employing the template-switch activity of the reverse transcriptase a linker sequence was added during the reverse transcription reaction to the 3' end of the cDNA through reverse transcribing a template switch oligo (Seq-1; Linker1) (US 5962271, US 5962372). Then the 5' end of the generated cDNA comprises a polyT-stretch introduced by the oligo which corresponds to the mRNA's original polyA-tail plus the Linker2 sequence. The 3' end of the cDNA comprises the reverse complement to the Linker1 sequence preceded by an additional C nucleotide that is added cap dependent. Two different sets of samples were prepared for sequencing.

The single sample of comparative set A (without segregation; 0 matrix) was prepared by PCR-amplifying in a 50 µl reaction about 27 pg of cDNA to a level of about 800 ng using primers that hybridize to the template switch sequence (Seq-3; Linker1) at the 3' end and the polyT sequence at the 5' end of the cDNA (Seq-4, Linker2-T₂₇). To generate enough material for the subsequent sequencing sample preparation, eight purified PCR reactions were mixed and about 5 µg were further processed. In its essence, this sample contained a non-specific matrix with only one field therefore representing an amplification where the whole cDNA could have served as a template.

Set B (with segregation) consists of 6 samples that correspond to 6 subpools of a 12 subpool matrix (1x1 matrix).

The expression "1x1 matrix" as used herein refers to 1 selective nucleotide at the 3' terminus of the cDNA and 1 selective nucleotide at the 5' terminus of the cDNA. For each selective nucleotide a segregation into pools for each of the four nucleotides is possible. However, if mRNA is used as template comprising a polyA-tail, the nucleotide next to the tail (or the corresponding polyT stretch on a cDNA) can only select for the other three nucleotides (thus this nucleotide can be used to segregate into 3 subpools). A 1x1 matrix for mRNA with a polyA-tail (segregating for terminal nucleic acid types, i.e. next to the tail) can therefore segregate into 4x3=12 subpools. For example other matrices such as a 2x0 matrix segregates into 4x4=16 subpools, a 0x2 matrix segregates into 3x4=12 subpools, or a 2x2 matrix segregates into 3x4x4x4=192 subpools.

To generate 12 subpools one of four primers with a 3'-terminal A, G, C or T specific for the 3' end of the cDNA and one of three primers with a 3'-terminal A, G or C specific for the 5' end of the cDNA was applied to selectively amplify in each matrix field only cDNA molecules with one specific termini combination. To generate the 6 samples (subpools) of set B only six 5'/3' (cDNA) primer combinations were used (Seq-9/Seq-5 (C/G); Seq-10/Seq-5 (G/G); Seq-11/Seq-6 (A/A); Seq-9/Seq-7 (C/C); Seq-10/Seq-7 (G/C)); Seq-11/Seq-8 (A/T)), each amplifying about 27pg of cDNA to 800 ng; 5 µg of 8 pooled replicates for each primer combination was used in subsequent reactions. In its essence, each of the 6 PCR samples of set B on average used a 1/12 of the cDNA as a template.

**Table 1:Oligonucleotides used in example 1 for reverse transcription of RNA and matrix PCR. The asterix depicts a phosphorothioate bond; ribonucleotides are preceded by an "r".**

| Seq-ID | Sequence |
|---|---|
| Seq-1 | A*CTGTAAAACGACGGCCAGTATAGTTATTGATATGTAATACGACTCACTATArG*rG*rG |
| Seq-2 | A*CGGAGCCTATCTATATGTTCTTGACATTTTTTTTTTTTTTTTTTTTTTTTTT*T*V |
| Seq-3 | G*TTATTGATATGTAATACGACTCACTAT*A |
| Seq-4 | G*ACATTTTTTTTTTTTTTTTTTTTTTTTTT^{*}T |
| Seq-5 | T*AATACGACTCACTATAGGGG^{*}G |
| Seq-6 | T*AATACGACTCACTATAGGGG*A |
| Seq-7 | T*AATACGACTCACTATAGGGG*C |
| Seq-8 | T*AATACGACTCACTATAGGGG*T |
| Seq-9 | N*NTTTTTTTTTTTTTTTTTTTTTTTTT*C |
| Seq-10 | N*NTTTTTTTTTTTTTTTTTTTTTTTTT*G |
| Seq-11 | N*NTTTTTTTTTTTTTTTTTTTTTTTTT*A |

To prepare the samples of the two sets for next generation sequencing, each of the PCR sample was fragmented (by sonication) into fragments which were on average 200-1000 bp long. Afterwards, the samples were subjected to a standard Illumina genomic DNA sequencing sample preparation pipeline using an Illumina Genomic Prep Kit (#FC-102-1001; Illumina Inc., USA). In essence, adapters were added to the ends of the fragments, which were used to bind the samples to the flow cell. They allow for cluster generation and enable the hybridization of a sequencing primer to start the sequencing run. In addition the 6 samples of set B were bar-coded with standard Illumina multiplex tags using the Multiplexing Sample Preparation Oligonucleotide Kit (#PE-400-2002; Illumina Inc., USA). Adapter ligated fragments in a size range of 200-600 bp were size selected for sequencing.

The single sample of set A was loaded onto one channel of the flow cell and the 6 samples of set B mixed in equal amounts and loaded onto a second channel. Cluster generation was carried out on a cBot Instrument (Illumina Inc., USA) using the Cluster generation Kit (#GD-203-2001, version 2; Illumina Inc., USA). Then a 76 bp sequencing run was carried out on a GenomeAnalyzer II (Illumina Inc.) using the Sequencing Reagent Kit (#FC-104-3002, version 3; Illumina Inc., USA).

The multiplex tags of the 6 samples of set B were read out using Multiplex Sequencing Primer and PhiX Control Kit (#PE400-2002,version 2; Illumina Inc., USA).

For each of the channels, short (76 bp) reads were obtained, and the multiplexed reads of set B were separated according to their barcodes.

Then the number of reads for both data sets was normalized by randomly drawing 4950084 reads for set A. For each of the six samples of set B 825014 reads where randomly drawn, therefore set B in total consisted of 4950084 reads.

For performing the bioinformatic analysis of the read sets the CLC Genomics Workbench V3.6.5 (CLC bio, Denmark) was used.

The 5' primer sequences were trimmed off the reads, all erroneous nucleotides (Ns) were clipped off the reads and reads below a threshold length of 20 nucleotides were excluded from further analysis.

The resulting 4940840 and 4948650 reads for sets A and B were used for subsequent analysis.

### a) Alignment to a reference mRNA database

The refMrna database was downloaded [1] on the 4^{th} of October 2009 from the UCSC Genome Browser webpage [6] and contains 24570 reference mRNA sequences that are based on the mouse genome assembly (mm9, NCBI built 37). In order to investigate how many of these reference mRNAs could be detected without and with segregation an alignment of read set A and read set B to these reference mRNAs was done. For both alignments the following CLC parameters were used (Add conflict annotations = No; Conflict resolution = Vote; Create Report = Yes; Create SequenceList = Yes; Match mode = random; Sequence masking = No; Similarity = 0,8; Length fraction = 0,5; Insertion cost = 3; Deletion cost = 3; Mismatch cost = 2). Set A (without segregation) detected 15652 mRNAs. An increase to 15702 detected mRNAs could be observed for data set B. As data set B contained only 6 out of 12 possible subpools this slight increase is significant.

However as the refMrna dataset contains only on the order of one transcript per known gene, an alignment of both sets to a more complete dataset that contains also more transcript variants of genes (e.g. splice variants) was carried out.

### b) Alignment to 328358 mRNA sequences

328358 GenBank mRNA sequences [5] were downloaded [2] on October 4^{th}, 2009 from the UCSC genomics browser database [6]. Applying the same CLC parameters as in a) set A and set B were aligned to these 328358 GenBank mRNA sequences. Using set A 83199 sequences could be detected and using set B 87794 sequences could be detected. This amounts to about 5% more mRNA molecules that could be detected when segregation was carried out before sequencing.

Though the observed improvement is significant, even this large mRNA databases is limited in both breadth (number of genes) and depth (transcript variants of a gene).

Therefore in addition alternative analyses were carried out in a genomic context.

### c) Assembly against the mouse genome

The complete reference mouse genome was downloaded [3] on the 4^{th} of October 2009 from UCSC genome browser database [6]. Alignments were made using the same CLC parameters as in a) and resulted in a genomic coverage of 0.494% for data set A and 0.561% for data set B (Fig. 10). Therefore, set B detects about 13.5% percent more of the genome compared to set A. This translates to about 1835663 additionally mapped nucleotides. If the mean exon size for mouse is about 300-400 bases then about 4589 to 6118 additional exons can be detected.

Furthermore, fig. 10 demonstrates that the read alignment results in increased genomic coverage independent of the read depth, and that the same genomic coverage can be obtained with less read depth when using a segregated sample (set B) compared to a non-segregated sample. In the analysis subclasses of reads were created by randomized drawing and then aligned separately to the reference genome. The difference in genomic coverage at 100 Mbp read depth is 20% and at 1 Gbp 30%.

### d) RNA-Seq analysis against the annotated mouse genome

Combining genomic and transcriptomic information a characterization of possible unknown exons within rather narrow borders of up to 1000 bases up and downstream of known genes was performed. Here, the complete annotated reference mouse genome that was downloaded from NCBI [4] database (NCBI Build 37, mm9, C57BL/6J, July 2007) was used as a reference. An RNA-Seq analysis [7] was carried out using again the CLC Genomics Workbench. The parameter set were modified in order to include 1000 nucleotides upand downstream of annotated gene sequences (Additional upstream bases = 1000; Additional downstream bases = 1000; Create list of unassembled reads = Yes; Exon discovery = Yes; Maximum number of mismatches (short reads) = 2; Minimum length of putative exons = 50; Minimum number of reads = 10; Organism type = Eukaryote; Unspecific match limit = 10; Use colorspace encoding = No; Use gene annotations = Yes; Expression value = RPKM; Minimum exon coverage fraction = 0,2; Minimum length fraction (long reads) = 0,9). Integration of data set A revealed 207 putatively novel exons of which at least 73 were uniquely detected by set A alone. Data set B improved these numbers markedly and yielded 256 putatively novel exons, at least 122 of which were discovered by B alone. Therefore segregation will reveal more novel information even in the context of known genes.

### e) Segregation of transcript variants of individual genes in the context of all genes

As in d) the annotated reference mouse genome was used to determine the expression values (RPKM) in an RNA-Seq analysis [7] using CLC Genomics Workbench. A comparison was carried out comparing the gene expression values between individual subpools and the combined 6 subpools.. A scatter plot comparing subpool 6 to the combined subpools is shown in figure 11.

As a random distribution would lead to a scatter around the 1/6 line, figure 11 clearly shows that segregation has occurred as the scatter is distributed across all six segments. This means that transcript variants of individual genes are segregated into different subpools in relation to their concentration in the sample. For instance, a gene that is drawn in above the 5/6 line has one or more transcript variants in this subpool that account for more than 5/6 of the concentration of all transcript variants of that gene.

A summary of the grouping according to the distribution of snRPKM values for all subpools is shown in table 2. The number of annotated genes in the genome NCBI data bank where in total 31781. In all 6 subpools together 11478 genes have been detected. Genes that are drawn in above the 6^{th} part are in summary 2688 or 23.4%. For these genes variation in concentration between samples in other subpools (meaning for other transcriptvariants) is harder to detect without segregation than with segregation.

**Table 2: Distribution of the sum normalized RPKM (snRPKM) values per subpool resulting from respective 0.825 Mio reads in relation to the sum normalized RPKM (snRPKM) values of entire 4.95 Mio reads of all 6 subpools.**

| | Subpool 1 | Subpool 2 | Subpool 3 | Subpool 4 | Subpool 5 | Subpool 6 |
|---|---|---|---|---|---|---|
| sixth part | | | | | | |
| 1. | 3,069 | 3,435 | 3,092 | 3,389 | 3,842 | 3,023 |
| 2. | 1,597 | 1,472 | 1,918 | 1,441 | 1,128 | 1,909 |
| 3. | 1,114 | 931 | 1,520 | 890 | 472 | 1,610 |
| 4. | 631 | 575 | 556 | 354 | 120 | 633 |
| 5. | 294 | 344 | 138 | 141 | 50 | 200 |
| 6. | 453 | 478 | 441 | 315 | 187 | 500 |
| 6.+ | 44 | 39 | 43 | 68 | 56 | 64 |
| total | 7,202 | 7,274 | 7,708 | 6,598 | 5,855 | 7,939 |

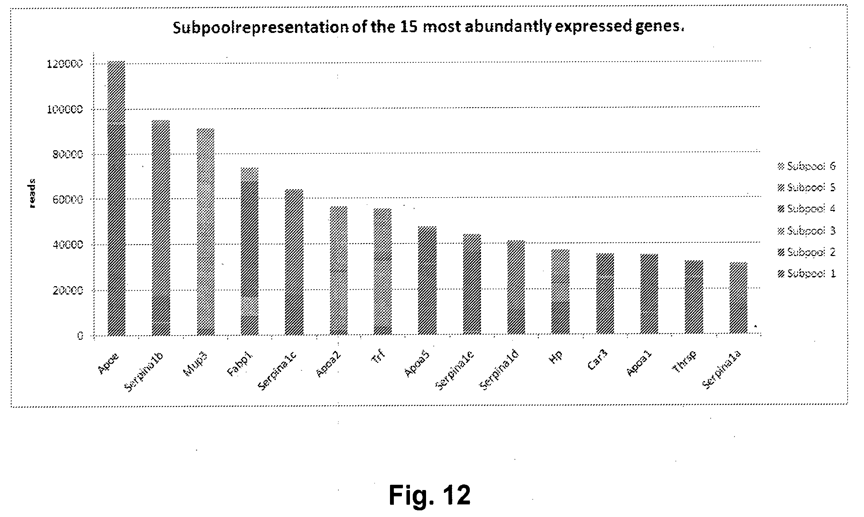

Furthermore the distribution of transcript variants into different subpools is different for each gene as shown exemplary in figure 12 for the subpool distribution of the 15 most abundant genes. This means that reads that map to the same gene and are found in different subpools belong to different transcript variants that are potentially differentially expressed.

### f) Segregation of transcript variants of a single gene into subpools

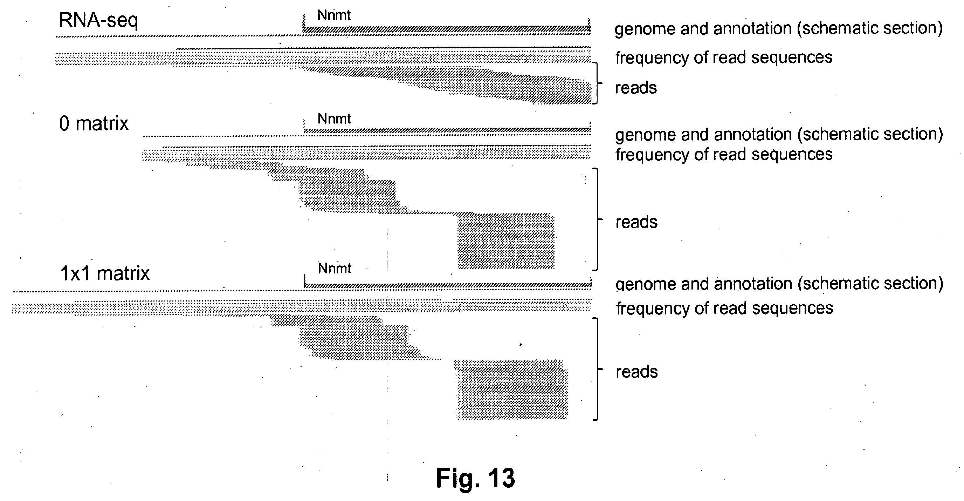

The consequences of such segregation for a single gene are shown in detail for one example, the nicotinamide N-methyltransferase gene (Nnmt: ENSMUSG00000032271). Nnmt carries currently two protein-coding mRNA annotations, ENSMUST00000034808 and ENSMUST00000119426, and 3 further annotations. In addition to a 0 matrix (Set A) and a 1x1 matrix (Set B) an 4.96 Mio. reads of an RNA-seq protocol [7] were used in the comparison.
First, using the RNA-seq protocol 185 reads could be mapped to the Nnmt gene of which none could be clearly attributed to a transcription start sequence (see figure 13). Neither the two protein-coding transcripts nor any other transcripts could be distinguished with confidence.

Second, a 0 matrix protocol (set A) mapped 3,266 reads resulting in a higher total RPKM value. Because of the linker sequence tag (Linker1), 105 reads were identified as start sequences. 11 different starting sites were mapped with a 2 reads threshold. The remaining 3,161 reads, that had no Linker1 tag and thus are internal reads, could not be assigned to any of those 11 different transcript variations because of the missing segregation.

Third, using a segregated sequencing library (set B), which corresponds to 6 out of 12 possible subpools, produced 3.680 reads, approximately the same number of reads as the 0-matrix above. 135 reads could be identified as start sequences. With the 2 reads threshold 9 different transcription start sites were identified. Therefore all reads that do not have the start site tag (Linker1) must belong to one of the mapped start site in the corresponding subpool.

Table 3 summarizes the detailed further analysis. The 9 start sites distribute across 4 of the 6 subpools. The number of start sites adds actually up to 11, but two of the start sites in related G/- and C/- subpools, G/G and G/C as well as C/C and C/G, are identical. By investigating the identified start sites the assignment to subsequent larger matrices was investigated. A 2x1 has only 5 different start sites remaining in just 2 subpools, GT/C and GT/G. Expanding those two subpools into a 3x1 matrix enables the complete segregation of all detected start sites into 11 individual subpools. Therefore at that stage not only the 135 start sequence reads can be completely segregated, but also the whole 3.680 reads can be unambiguously assigned to the identified transcription start sites. This shows that increasing the number of selective subpools also increases the segregation power of a matrix.

**Table 3: 5'-start site analysis of Nmnt assigned 135 start site reads in a 1x1 matrix. The extrapolation of the subsequent 2x1 and 3x1 matrices reveal that the complete start site segregation can be achieved with a 3x1 matrix. ΣTS(2+)/ Σ, sum of transcription start site that are detected by 2 or more reads to the sum of all reads.**

| **1x1 matrix (set A)** | | **2x1 matrix (projected)** | | **3x1 matrix (projected)** | |
|---|---|---|---|---|---|
| **Selective nucleotide 5'/3' terminus** | **ETS (2+) / Σ** | **Selective nucleotide 5'/3' terminus** | **ETS (2+) / Σ** | **Selective nucleotide 5'/3' terminus** | **ETS (2+) / Σ** |
| **G/C** | 2/496 | **GA/C** | | | |
| | | **GC/C** | 1 | | |
| | | **GG/C** | 1 | | |
| | | **GT/C** | | | |
| **G/G** | 3/1872 | **GA/G** | 1 | | |
| | | **GC/G** | 1 | | |
| | | **GG/C** | 1 | | |
| | | **GT/G** | | | |
| **A/A** | 0/94 | | | | |
| **C/C** | 3/330 | **CA/C** | 1 | | |
| | | **CC/C** | | | |
| | | **CG/C** | | | |
| | | **CT/C** | 2 | **CTA/C** | |
| **C/G** | 3/794 | **CA/G** | | **CTC/C** | |
| | | **CC/G** | | **CTG/C** | 1 |
| | | **CG/C** | | **CTT/C** | 1 |
| | | **CT/G** | 3 | **CTA/G** | |
| | | | | **CTC/G** | 1 |
| | | | | **CTG/G** | 1 |
| | | | | **CTT/G** | 1 |
| **T/A** | 0/94 | | | | |

In conclusion, experiment 1 shows that segregating mRNAs employing even a small matrix (12 subpools) and furthermore using only half of such a matrix (6 out of 12 subpools) the detection of mRNAs significantly improves in a genomic as well as transcriptomic context.
[1]http://hgdownload.cse.ucsc.edu/goldenPath/mm9/bigZips/refMrna .fa.gz
[2]http://hgdownload.cse.ucsc.edu/goldenPath/mm9/bigZips/mrna.fa .gz
[3]http://hgdownload.cse.ucsc.edu/goldenPath/mm9/bigZips/chromFa .tar.gz
[4]http://www.ncbi.nlm.nih.gov/.
[5] Benson, Dennis A. ; Karsch-Mizrachi, Ilene ; Lipman, David J. ; Ostell, James ; Sayers, Eric W.: GenBank. In: Nucleic Acids Res 37 (2009) Nr. Database issue, S. D26-31
[6] Kuhn, R. M. ; Karolchik, D. ; Zweig, A. S. ; Wang, T. ; Smith, K. E. ; Rosenbloom, K. R. ; Rhead, B. ; Raney, B. J. ; Pohl, A. ; Pheasant, M. ; Meyer, L. ; Hsu, F. ; Hinrichs, A. S. ; Harte, R. A. ; Giardine, B. ; Fujita, P. ; Diekhans, M. ; Dreszer, T. ; Clawson, H. ; Barber, G. P. ; Haussler, D. ; Kent, W. J.: The UCSC Genome Browser Database: update 2009. In: Nucleic Acids Res 37 (2009) Nr. Database issue, S. D755-61
[7] Mortazavi, Ali ; Williams, Brian A. ; McCue, Kenneth ; Schaeffer, Lorian ; Wold, Barbara: Mapping and quantifying mammalian transcriptomes by RNA-Seq. In: Nat Methods 5 (2008) Nr. 7, S. 621-8

### Example 2: cDNA segregation through selective precipitation and downstream NGS.

In a first step, purified mRNA of a tissue sample becomes reverse transcribed and pre-amplified. In a second step, the pre-amplified cDNA is precipitated into different fractions by increasing PEG concentrations [8]. By this means, 10 pools are prepared which contain cDNA with different solubility. The solubility is manly influenced through the length of the cDNA.

The cDNA of the 10 different sub-pools is processed separately which involves fragmentation and labeling of each subpool with a sub-pool specific sequence tag. All fragments are transferred to the NGS platform and sequenced, reading out in addition the tag.

The reads are segregated according to the 10 different subpool tags. Now, in a first assembly contigs are built by aligning reads within each subpool. In comparison, in a second assembly contigs are built neglecting the sub-pool information. More and longer contigs can be assembled using in the first assembly when contig building was done within each subpool, compared to the second assembly, where the reads where not separated into subpools.
[8] Lis, John ; Size fractionation of double-stranded DNA by precipitation with polyethylene glycol. Nucleic Acids Research, volume 2 number 3 March 1975

### Example 3: mRNA segregation through size separation and downstream NGS

10µg of mRNA of a tissue sample is electrophoretically separated on an agarose gel. After the densitometric characterization of the gel picture 12 bands are cut out. The bands hold about the same amount of mRNA. Each band is defined through one lower and one higher cut-off length according to the weight marker. The bands segregate all mRNA according to 1) 25-100bp, 2) 100-500bp, ... 12) 12000-∞bp. The mRNA is purified from the gel bands, prepared separately for NGS sequencing adding a sequence tag to each of the 12 subpools. The 12 tagged subpools are mixed in equal amounts and sequenced in one lane on an Illumina Genome Analyzer II instrument.

The NGS provides 12 times 0.8 Mio reads. Now, in a first analysis the reads are aligned to each other under guidance of the known consensus genome with the aim to construct complete transcripts. Transcripts not only have to oblige the sequence matches, in addition, each transcript must have a certain lower length and is not allowed to exceed a maximum length with respect to its band size sub-pool. In comparison, a second alignment is done neglecting the sub-pool and size information. In comparison the mean contig length of the first alignment is higher and the first alignment contains more full length sequences than the second alignment.

### Example 4: Computerized calculation of improvement.

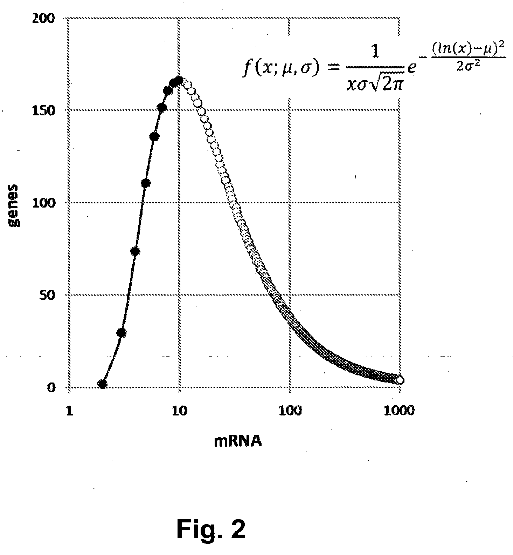

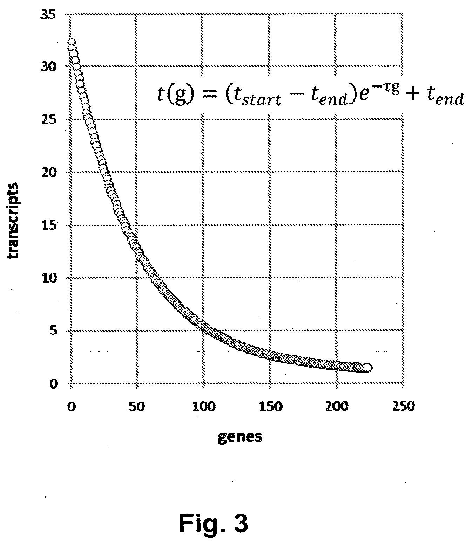

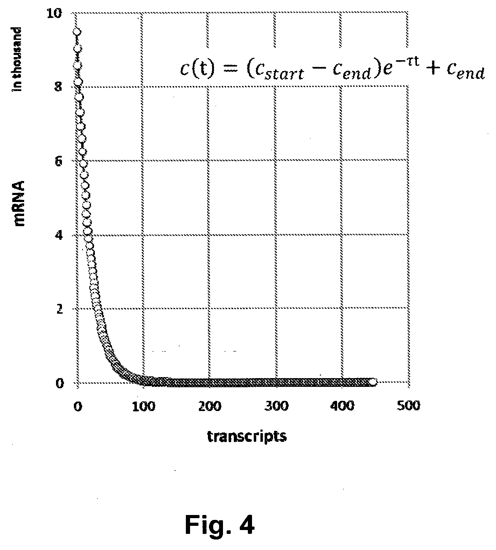

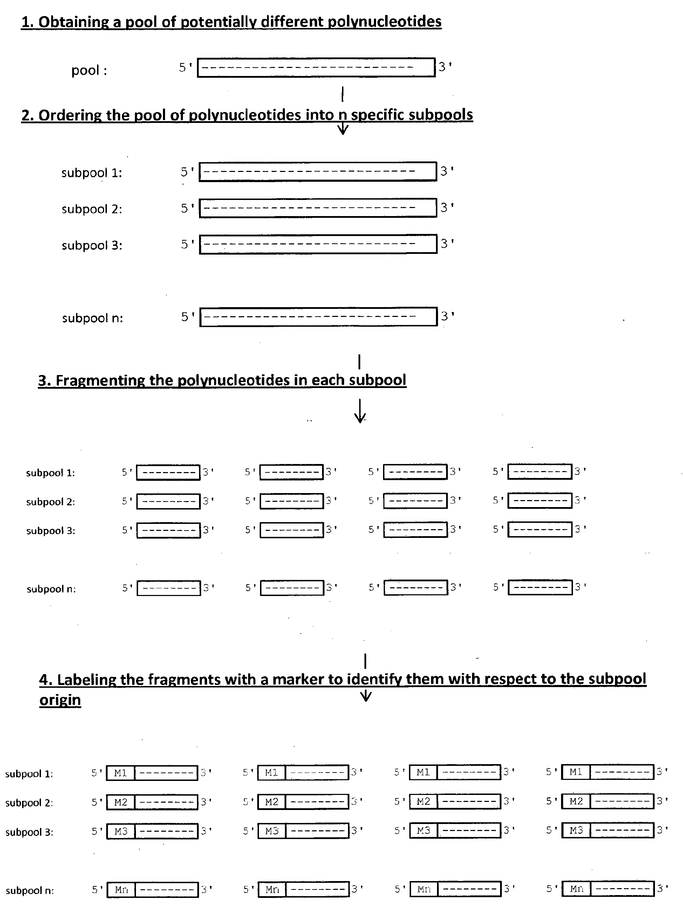

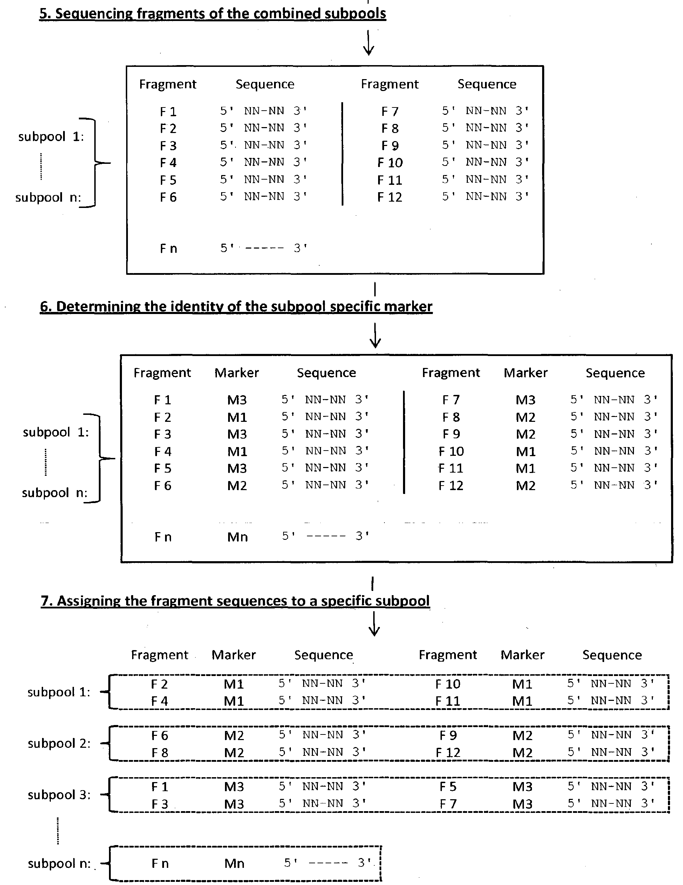

Random sequences were generated using a Random Letter Sequence Generator (http://www.dave-reed.com/Nifty/randSeq.html) and arranged in a data base, e.g. because of the small size it could be done in a spreadsheet, assembling the genes of the model genome. All randomized numbers (e.g. gene and number of transcripts) were generated using a randomizer. Then, the genes were used to generate the model transcriptome according to the statistical requirements illustrated trough the graphs in fig.2 to 4. Their total number is listed in column "trans" in table 5. To simplify matter, all transcripts are complete copies of their parental gene, so no variants are introduced yet.

For experiment 5 just 10 short genes (10 gene genome in tab. 1) have been chosen to illustrate the underlying principles in a simplistic manner.

**Table 4: Short randomized sequences used as pool model.**

| gene | length | Sequence |
|---|---|---|
| 1 | 202 | |
| 2 | 242 | |
| 3 | 275 | |
| 4 | 297 | |
| 5 | 305 | |
| 6 | 297 | |
| 7 | 275 | |
| 8 | 242 | |
| 9 | 202 | |
| 10 | 160 | |

First, the transcripts were ordered into 16 (4×4) different pools according to their terminal bases (table 6).

Because one particular transcriptome (all reads align to the blue print) is selected and any reading errors are excluded, a simple alignment algorithm (simple search function which provides the number of sequence matches) could be used to probe the genome/transcriptome. It selects all reads that have a perfect k-mer match to the reference sequence (transcriptome). So, 24 permutations of 4bp fragments (without any base repeats like AATG) were taken and aligned, once against the entire model genome/transcriptome (tab. 5) and once against the segregated genome/transcriptome (tab. 6). The number of unique hits is shown in both tables in the right column.

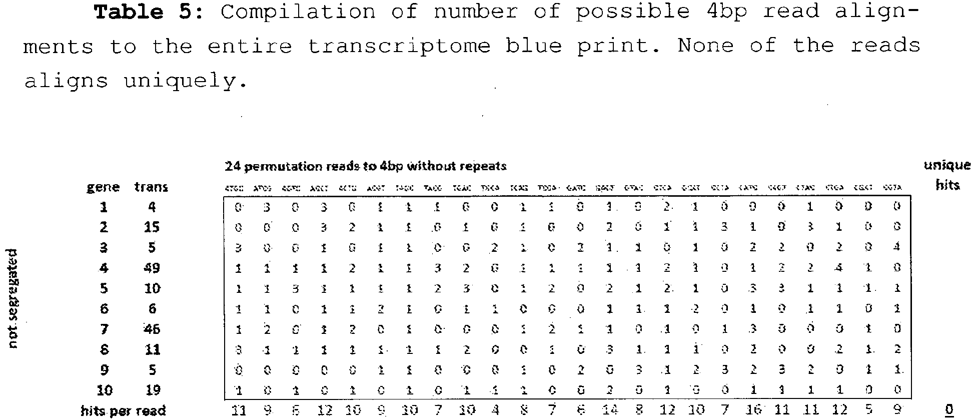

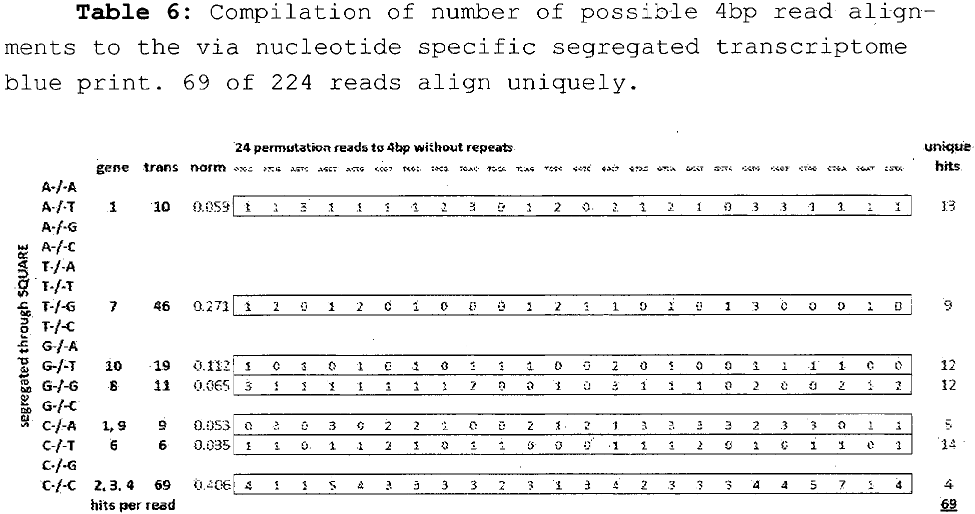

This example shows, that
i) none of the 24 probed reads gave one unique hit when trying to align reads to the entire genome/transcriptome. The number of total hits was 224. The most unique read aligned matched 4 different genes/transcripts.
ii) After segregation into 7 sub-pools, here according to the molecule ends, 69 (31%) of the reads could already be aligned uniquely.

Even without having a blue print the same principle applies. In the first case none of the investigated reads will belong to a unique position in the pool, whereas 31% of the reads will have one unique position in their host sub-pool. The relative value regarding the alignment within the pool of transcripts is given by the number in the column "norm" in table 6. For example, the 4 unique hits in pool C-/-C containing the highly abundant transcripts of genes 20, 30 and 40 do uniquely identify close to 40 percent of all transcripts.

### REFERENCES:

Liang, P. and A. B. Pardee. (1992) Differential display of eukaryotic messenger RNA by means of the polymerase chain reaction. Science, 257, 967-71.
Maruyama, K. and Sugano, S. (1994) Oligo-capping: a simple method to replace the cap structure of eukaryotic mRNAs with oligoribonucleotides. Gene, 138, 171 - 174.
Matz, M. et al., (1997) Ordered differential display: a simple method for systematic comparison of gene expression profiles. Nucleic Acids Res., 25, 2541-2542.
Shiraki, T., Kondo, S., Katayama, S., Waki, K., Kasukawa, T., Kawaji, H., Kodzius, R., Watahiki, A., Nakamura, M., Arakawa, T., Fukuda, S., Sasaki, D., Podhajska, A., Harbers, M., Kawai, J., Carninci, P. and Hayashizaki, Y. (2003) Cap analysis gene expression for high-throughput analysis of transcriptional starting point and identification of promoter usage. Proc Natl Acad Sci U S A, 100, 15776-81.
Nagalakshmi U. et al., Science, 320 (5881) (2008): 1344-1349
Armour C. D. et al., Nature Methods, 6 (9) (2009): 647
Breyne P. et al., MGG Mol. Genet. Genom., 269 (2) (2003): 173-179
Wilhelm B. T. et al., Methods, 48 (3) (2009): 249-257

## Claims

1. Method of ordering nucleic acid molecule fragment sequences derived from a pool of potentially diverse RNA molecules comprising
optionally reverse transcribing the RNA molecules to provide a pool of cDNA molecules,
segregating nucleic acids from said template RNA or cDNA pool by selecting for potentially different templates with a distinctive nucleic acid feature shared by the segregated templates, comprising selecting for potentially different templates with at least one given nucleotide type shared by the segregated templates at a certain position being i) within 100 nucleotides from either the 5' or 3' terminus of the full length template nucleic acid molecule sequence or ii) selected from the 1 to 100 nucleotides next to a polyA-tail or next to a polyT-tail of a cDNA or iii) selected from the 1 to 100 nucleotides next to a tail artificially attached to the template RNA or cDNA, and wherein said selecting is with a primer or probe, which is specific for said at least one distinctive nucleotide i), ii) or iii), thereby providing at least a first subpool of nucleic acids,
optionally once or more further segregating nucleic acids from said template RNA or cDNA, selectively segregating nucleic acids with a different distinctive nucleic acid feature, thereby providing one or more further subpool(s) of nucleic acids,
generating fragments of said segregated nucleic acid molecules by
a) random fragmenting or
b) obtaining fragment copies of said segregated nucleic acid molecules, and
attaching linkers or adaptors to the generated fragments,
wherein the fragments of each subpool or combined subpools remain separable from fragments of other subpools or other combined subpools by physically separating the subpools or by attaching a label to the fragments of the subpools, with the label identifying a subpool.

2. The method of claim 1 further comprising determining the sequence or partial sequence of the fragments of the first subpool and optionally further subpools, preferably wherein a partial sequence of at least 10, in particular preferred at least 18, even more preferred at least 25, nucleotides is determined.

3. The method of claim 1 or 2, **characterized in that** the RNA molecules are of a biological sample, preferably of a virus, prokaryote or eukaryote.

4. The method of any one of claims 1 to 3, **characterized in that** random fragmenting the segregated nucleic acid molecules comprises random fragmenting by physical means, in particular preferred by shearing, sonication or elevated temperatures; or obtaining fragment copies of said segregated nucleic acid molecules comprises generating fragments by amplification in a sequence independent or sequence dependent method, preferably by random priming.

5. Method of ordering nucleic acid molecule fragment sequences derived from a pool of potentially diverse RNA molecules comprising
· optionally reverse transcribing the RNA molecules to provide a pool of cDNA molecules,
· segregating nucleic acids from said template RNA or cDNA pool by selecting for potentially different templates with a distinctive nucleic acid feature shared by the segregated templates, comprising selecting for potentially different templates with at least one given nucleotide type shared by the segregated templates at a certain position being i) within 100 nucleotides from either the 5' or 3' terminus of the full length template nucleic acid molecule sequence or ii) selected from the 1 to 100 nucleotides next to a polyA-tail or next to a polyT-tail of a cDNA or iii) selected from the 1 to 100 nucleotides next to a tail artificially attached to the template RNA or cDNA, and wherein said selecting is with a primer or probe, which is specific for said at least one distinctive nucleotide i), ii) or iii), thereby providing at least a first subpool of nucleic acids,
· optionally once or more further segregating nucleic acids from said template RNA or cDNA, selectively segregating nucleic acids with a different distinctive nucleic acid feature, thereby providing one or more further subpool(s) of nucleic acids,
· generating fragments of said segregated nucleic acid molecules by determining a partial sequence of said segregated nucleic acid molecule by a nano-pore.

6. The method of any one of claims 1 to 5, **characterized in that** the fragments consist of 10 to 10000 nucleotides, preferably of 25 to 500 nucleotides.

7. The method of any one of claims 1 to 6, **characterized in that** the nucleic acid feature is a given nucleotide type, preferably selected from any one of A, T, U, G, C, at a certain position in the nucleic acid molecule, preferably the position being within 100 nucleotides from either the 5' or 3' terminus of the nucleic acid molecule, especially preferred wherein the nucleic acids are selected for common nucleotides within the 10 nucleotides next to the 5' and/or 3' terminus, preferably for one or more common 5' and/or 3' terminal nucleotide types.

8. The method of any one of claims 1 to 7, **characterized in that** said RNA molecule is a full length RNA and/or the segregated nucleic acid molecule comprises the sequence of the full length or complete cDNA or RNA.

9. The method of claim 2, **characterized in that** sequence determinations comprises determining the sequence of at least 5, preferably at least 8, nucleotides from the fragment, in particular from either its 5' or the 3' end, even more preferred determining the full sequence of the fragment.

10. The method of any one of claims 1 to 9, **characterized in that** the nucleic acids are divided into subpools wherein at least 10% of all subpools comprise the average amount of nucleic acids of all subpools +/-50%.

11. The method of any one of claims 1 to 10, **characterized in that** the nucleic acids are divided into subpools wherein at least 10% of the subpools contain 2 or less nucleic acids, preferably 1 nucleic acid.

12. The method of any one of claims 1 to 11, **characterized in that** segregating nucleic acids comprises specifically amplifying nucleic acids from said template pool, preferably wherein the amplification is performed by nucleotide extension from a primer, preferably by PCR, in particular preferred wherein the amplification is performed by using primers which select for at least one, preferably at least two, in particular at least two adjacent, different nucleotides after an unspecific primer portion whereby nucleic acid molecules are amplified which comprise the selected nucleotide as the nucleic acid feature specific for a subpool.

13. The method of any one of claims 1 to 12, **characterized by** attaching a subpool-specific label to the fragments, preferably wherein the subpool-specific label is one or more nucleotides, which are preferably co-determined during sequence determination as defined in claim 2.

14. The method of any one of claims 1 to 13, further comprising amplifying the nucleic acid molecules, preferably after segregation, prior to determining the sequence, in particular preferred wherein said amplification is by PCR and at least one nucleotide molecule is amplified to.the saturation phase of the PCR, in particular preferred at least 10% of the different nucleotide molecules are amplified to the saturation phase of the PCR.

15. The method of any one of claims 1 to 14, **characterized in that** subpools with high abundant nucleic acid molecules are excluded from sequence determination, wherein subpools with high abundant nucleic acids molecules are subpools comprising more than 1000% nucleic acid molecules above the average amount of all subpools.

16. The method of any one of claims 1 to 15, **characterized in that** during segregation of the nucleic acid one selected strand is segregated or one selected strand is labeled, wherein preferably the fragments of the selected strand are also labeled.

## Patentansprüche

1. Verfahren zum Ordnen von Nukleinsäuremolekülfragmentsequenzen, die aus einem Pool von potentiell unterschiedlichen RNA-Molekülen abgeleitet sind, umfassend
• gegebenenfalls das reverse Transkribieren der RNA-Moleküle, um einen Pool von cDNA-Molekülen bereitzustellen,
• das Isolieren von Nukleinsäuren aus dem Template-RNA- oder cDNA-Pool anhand des Selektierens nach potentiell unterschiedlichen Templates mit einem distinktiven Nukleinsäuremerkmal, das die isolierten Templates gemeinsam aufweisen, umfassend das Selektieren nach potentiell unterschiedlichen Templates, wobei die isolierten Templates mindestens eine vorgegebene Nukleotidart gemeinsam aufweisen, und zwar an einer bestimmten Position i) innerhalb von 100 Nukleotiden von entweder dem 5'- oder dem 3'-Terminus der Volllängensequenz des Template-Nukleinsäuremoleküls oder ii) ausgewählt aus den Nukleotiden 1 bis 100 im Anschluss an einen poly A-Schwanz oder im Anschluss an einen poly T-Schwanz einer cDNA oder iii) ausgewählt aus den Nukleotiden 1 bis 100 im Anschluss an einen künstlich an der Template-RNA oder -cDNA befestigten Schwanz, und wobei das Selektieren mittels eines Primers oder einer Sonde erfolgt, der bzw. die spezifisch für mindestens ein distinktives Nukleotid gemäß i), ii) oder iii) ist, wodurch mindestens ein erster Subpool von Nukleinsäuren bereitgestellt wird,
• gegebenenfalls das ein- oder mehrmalige weitere Isolieren von Nukleinsäuren aus der Template-RNA oder -cDNA unter selektiver Isolation von Nukleinsäuren mit einem unterschiedlichen distinktiven Nukleinsäuremerkmal, wodurch ein oder mehrere weitere Subpools von Nukleinsäuren bereitgestellt werden,
• das Erzeugen von Fragmenten der isolierten Nukleinsäuremoleküle durch
a) randomisiertes Fragmentieren oder
b) den Erhalt von 2Fragmentkopien der isolierten Nukleinsäuremoleküle, und
das Befestigen von Linkern oder Adaptern an den erzeugten Fragmenten, wobei die Fragmente aus jedem der Subpools oder aus kombinierten Subpools in einem Zustand verbleiben, in dem sie von Fragmenten aus weiteren Subpools oder weiteren kombinierten Subpools getrennt werden können, und zwar entweder durch das physikalische Trennen der Subpools oder durch das Anbringen einer Markierung an den Fragmenten der Subpools, wobei der Subpool durch die Markierung gekennzeichnet ist.

2. Verfahren nach Anspruch 1, des Weiteren umfassend das Bestimmen der Sequenz oder Teilsequenz der Fragmente des ersten Subpools und gegebenenfalls weiterer Subpools, wobei bevorzugt eine Teilsequenz von mindestens 10, insbesondere bevorzugt mindestens 18, noch mehr bevorzugt mindestens 25, Nukleotiden bestimmt wird.

3. Verfahren nach Anspruch 1 oder 2, **dadurch gekennzeichnet, dass** die RNA-Moleküle von einer biologischen Probe stammen, bevorzugt von einem Virus, Prokaryonten oder Eukaryonten.

4. Verfahren nach einem der Ansprüche 1 bis 3, **dadurch gekennzeichnet, dass** das randomisierte Fragmentieren der isolierten Nukleinsäuremoleküle das randomisierte Fragmentieren anhand physikalischer Mittel umfasst, insbesondere bevorzugt durch Scheren, Ultraschallbehandlung oder erhöhte Temperaturen; oder dass der Erhalt von Fragmentkopien der isolierten Nukleinsäuremoleküle das Erzeugen von Fragmenten mittels Amplifikation mittels eines sequenzunabhängigen oder sequenzabhängigen Verfahrens, bevorzugt durch randomisiertes Priming.

5. Verfahren zum Ordnen von Nukleinsäuremolekülfragmentsequenzen, die aus einem Pool von potentiell unterschiedlichen RNA-Molekülen abgeleitet sind, umfassend
• gegebenenfalls das reverse Transkribieren der RNA-Moleküle, um einen Pool von cDNA-Molekülen bereitzustellen,
• das Isolieren von Nukleinsäuren aus dem Template-RNA- oder cDNA-Pool anhand des Selektierens nach potentiell unterschiedlichen Templates mit einem distinktiven Nukleinsäuremerkmal, das die isolierten Templates gemeinsam aufweisen, umfassend das Selektieren nach potentiell unterschiedlichen Templates, wobei die isolierten Templates mindestens eine vorgegebene Nukleotidart gemeinsam aufweisen, und zwar an einer bestimmten Position i) innerhalb von 100 Nukleotiden von entweder dem 5'- oder dem 3'-Terminus der Volllängensequenz des Template-Nukleinsäuremoleküls oder ii) ausgewählt aus den Nukleotiden 1 bis 100 im Anschluss an einen poly A-Schwanz oder im Anschluss an einen poly T-Schwanz einer cDNA oder iii) ausgewählt aus den Nukleotiden 1 bis 100 im Anschluss an einen künstlich an der Template-RNA oder -cDNA befestigten Schwanz, und wobei das Selektieren mittels eines Primers oder einer Sonde erfolgt, der bzw. die spezifisch für mindestens ein distinktives Nukleotid gemäß i), ii) oder iii) ist, wodurch mindestens ein erster Subpool von Nukleinsäuren bereitgestellt wird,
• gegebenenfalls das ein- oder mehrmalige weitere Isolieren von Nukleinsäuren aus der Template-RNA oder -cDNA unter selektiver Isolation von Nukleinsäuren mit einem unterschiedlichen distinktiven Nukleinsäuremerkmal, wodurch ein oder mehrere weitere Subpools von Nukleinsäuren bereitgestellt werden,
• das Erzeugen von Fragmenten der isolierten Nukleinsäuremoleküle durch das Bestimmen einer Teilsequenz des isolierten Nukleinsäuremoleküls mittels einer Nanopore.

6. Verfahren nach einem der Ansprüche 1 bis 5, **dadurch gekennzeichnet, dass** die Fragmente aus 10 bis 10.000 Nukleotiden, bevorzugt aus 25 bis 500 Nukleotiden, bestehen.

7. Verfahren nach einem der Ansprüche 1 bis 6, **dadurch gekennzeichnet, dass** es sich bei dem Nukleinsäuremerkmal um eine vorgegebene Nukleotidart, bevorzugt ausgewählt aus einer beliebigen von A, T, U, G, C, an einer bestimmten Position in dem Nukleinsäuremolekül handelt, wobei die Position bevorzugt innerhalb von 100 Nukleotiden von entweder dem 5'- oder dem 3'-Terminus des Nukleinsäuremoleküls liegt, wobei die Nukleinsäuren insbesondere bevorzugt nach gemeinsamen Nukleotiden innerhalb der 10 Nukleotide im Anschluss an den 5'- und/oder 3'-Terminus, bevorzugt nach einer oder mehreren gemeinsamen 5'- und/oder 3'-terminalen Nukleotidarten, selektiert werden.

8. Verfahren nach einem der Ansprüche 1 bis 7, **dadurch gekennzeichnet, dass** es sich bei dem RNA-Molekül um eine RNA voller Länge handelt und/oder das isolierte Nukleinsäuremolekül die Sequenz der Volllängen-cDNA oder -RNA bzw. der kompletten cDNA oder RNA umfasst.

9. Verfahren nach Anspruch 2, **dadurch gekennzeichnet, dass** die Sequenzbestimmungen das Bestimmen der Sequenz von mindestens 5, bevorzugt mindestens 8, Nukleotiden des Fragments, insbesondere ausgehend von entweder dessen 5'- oder 3'-Terminus, noch mehr bevorzugt das Bestimmen der vollständigen Sequenz des Fragments, umfassen.

10. Verfahren nach einem der Ansprüche 1 bis 9, **dadurch gekennzeichnet, dass** die Nukleinsäuren in Subpools unterteilt sind, wobei mindestens 10% aller Subpools die durchschnittliche Menge an Nukleinsäuren aller Subpools ± 50% umfassen.

11. Verfahren nach einem der Ansprüche 1 bis 10, **dadurch gekennzeichnet, dass** die Nukleinsäuren in Subpools unterteilt sind, wobei mindestens 10% der Subpools 2 oder weniger Nukleinsäuren, bevorzugt 1 Nukleinsäure, enthalten.

12. Verfahren nach einem der Ansprüche 1 bis 11, **dadurch gekennzeichnet, dass** das Isolieren von Nukleinsäuren das spezifische Amplifizieren von Nukleinsäuren aus dem Templatepool umfasst, wobei die Amplifikation bevorzugt mittels Nukleotidextension ausgehend von einem Primer, bevorzugt mittels PCR, erfolgt, wobei die Amplifikation insbesondere bevorzugt anhand der Verwendung von Primern erfolgt, die nach mindestens einem, bevorzugt nach mindestens zwei, insbesondere mindestens zwei benachbarten, unterschiedlichen Nukleotiden im Anschluss an einen unspezifischen Primerabschnitt selektieren, wodurch die Nukleinsäuremoleküle amplifiziert werden, die das selektierte Nukleotid als das für einen Subpool spezifische Nukleinsäuremerkmal umfassen.

13. Verfahren nach einem der Ansprüche 1 bis 12, **dadurch gekennzeichnet, dass** eine subpoolspezifische Markierung an den Fragmenten befestigt wird, wobei es sich bei der subpoolspezifischen Markierung um eines oder mehrere Nukleotide handelt, die bevorzugt im Verlauf der wie in Anspruch 2 definierten Sequenzbestimmung gemeinsam bestimmt werden.

14. Verfahren nach einem der Ansprüche 1 bis 13, des Weiteren umfassend das Amplifizieren der Nukleinsäuremoleküle, bevorzugt nach dem Isolieren, vor dem Bestimmen der Sequenz, wobei die Amplifikation insbesondere bevorzugt mittels PCR erfolgt und mindestens ein Nukleotidmolekül bis zur Sättigungsphase der PCR amplifiziert wird, insbesondere bevorzugt mindestens 10% der unterschiedlichen Nukleotidmoleküle bis zur Sättigungsphase der PCR amplifiziert werden.

15. Verfahren nach einem der Ansprüche 1 bis 14, **dadurch gekennzeichnet, dass** Subpools mit einem großen Überschuss an Nukleinsäuremolekülen von der Sequenzbestimmung ausgeschlossen sind, wobei es sich bei Subpools mit einem großen Überschuss an Nukleinsäuremolekülen um Subpools handelt, die mehr als 1.000 % an Nukleinsäuremolekülen über der durchschnittlichen Menge aller Subpools umfassen.

16. Verfahren nach einem der Ansprüche 1 bis 15, **dadurch gekennzeichnet, dass** im Verlauf des Isolierens der Nukleinsäure ein selektierter Strang isoliert wird oder ein selektierter Strang markiert wird, wobei die Fragmente des selektierten Strangs bevorzugt ebenfalls markiert werden.

## Revendications

1. Procédé permettant de classer des séquences de fragments de molécules d'acide nucléique dérivées d'un groupe de molécules d'ARN potentiellement différentes comprenant
- optionnellement la transcription inverse des molécules d'ARN pour fournir un groupe de molécules d'ADNc,
- la séparation des acides nucléiques à partir dudit groupe d'ARN ou d'ADNc matrices en sélectionnant des matrices potentiellement différentes avec une caractéristique d'acide nucléique distinctive partagée par les matrices séparées, comprenant la sélection de matrices potentiellement différentes avec au moins un type donné de nucléotide partagé par les matrices séparées au niveau d'une certaine position qui est i) au sein de 100 nucléotides de l'extrémité soit 5' soit 3' de la séquence de la molécule d'acide nucléique matrice pleine longueur ou ii) sélectionné parmi les 1 à 100 nucléotides à côté d'une queue polyA ou à côté d'une queue polyT d'un ADNc ou iii) sélectionné parmi les 1 à 100 nucléotides à côté d'une queue fixée artificiellement à l'ARN ou ADNc matrice, et où ladite sélection est réalisée avec une amorce ou une sonde, qui est spécifique dudit au moins un nucléotide distinctif i), ii) ou iii), fournissant de cette façon au moins un premier sous-groupe d'acides nucléiques,
- optionnellement une ou plusieurs autres séparations d'acide nucléiques à partir dudit ARN ou ADNc matrice, séparant sélectivement des acides nucléiques avec une caractéristique d'acide nucléique distinctive différente, fournissant de cette façon un ou plusieurs autres sous-groupe(s) d'acides nucléiques,
- la production de fragments desdites molécules d'acide nucléique séparées par
a) une fragmentation aléatoire ou
b) l'obtention de copies de fragments desdites molécules d'acide nucléique séparées, et
- la fixation de lieurs ou d'adaptateurs aux fragments produits, où les fragments de chaque sous-groupe ou de sous-groupes combinés demeurent séparables de fragments d'autres sous-groupes ou d'autres sous-groupes combinés par une séparation physique des sous-groupes ou par fixation d'un marqueur aux fragments des sous-groupes, avec le marqueur identifiant un sous-groupe.

2. Procédé selon la revendication 1, comprenant en outre la détermination de la séquence ou de la séquence partielle des fragments du premier sous-groupe et éventuellement d'autres sous-groupes, de préférence où une séquence partielle d'au moins 10, de façon particulièrement préférée d'au moins 18, encore plus préférentiellement d'au moins 25, nucléotides est déterminée.

3. Procédé selon la revendication 1 ou 2, **caractérisé en ce que** les molécules d'ARN sont d'un échantillon biologique, de préférence d'un virus, d'un procaryote ou d'un eucaryote.

4. Procédé selon l'une quelconque des revendications 1 à 3, **caractérisé en ce que** la fragmentation aléatoire des molécules d'acide nucléique séparées comprend une fragmentation aléatoire par des moyens physiques, de façon particulièrement préférée par cisaillement, sonication ou des températures élevées ; ou l'obtention de copies de fragments desdites molécules d'acide nucléique séparées comprend la production de fragments par amplification dans un procédé indépendant de la séquence ou dépendant de la séquence, de préférence par amorçage aléatoire.

5. Procédé de classement de séquences de fragments de molécules d'acide nucléique dérivées d'un groupe de molécules d'ARN potentiellement diverses comprenant
- optionnellement la transcription inverse des molécules d'ARN pour fournir un groupe de molécules d'ADNc,
- la séparation des acides nucléiques à partir dudit groupe d'ARN ou d'ADNc matrices en sélectionnant des matrices potentiellement différentes avec une caractéristique d'acide nucléique distinctive partagée par les matrices séparées, comprenant la sélection de matrices potentiellement différentes avec au moins un type donné de nucléotide partagé par les matrices séparées au niveau d'une certaine position qui est i) au sein de 100 nucléotides de l'extrémité soit 5' soit 3' de la séquence de la molécule d'acide nucléique matrice pleine longueur ou ii) sélectionné parmi les 1 à 100 nucléotides à côté d'une queue polyA ou à côté d'une queue polyT d'un ADNc ou iii) sélectionné parmi les 1 à 100 nucléotides à côté d'une queue fixée artificiellement à l'ARN ou l'ADNc matrice, et où ladite sélection est réalisée avec une amorce ou une sonde, qui est spécifique dudit au moins un nucléotide distinctif i), ii) ou iii), fournissant de cette façon au moins un premier sous-groupe d'acides nucléiques,
- optionnellement une ou plusieurs autres séparations d'acides nucléiques à partir dudit ARN ou ADNc matrice, séparant sélectivement des acides nucléiques avec une caractéristique d'acide nucléique distinctive différente, fournissant de cette façon un ou plusieurs autres sous-groupes d'acides nucléiques,
- la production de fragments desdites molécules d'acide nucléique séparées par la détermination d'une séquence partielle de ladite molécule d'acide nucléique séparée par un nanopore.

6. Procédé selon l'une quelconque des revendications 1 à 5, **caractérisé en ce que** les fragments sont constitués de 10 à 10 000 nucléotides, de préférence de 25 à 500 nucléotides.

7. Procédé selon l'une quelconque des revendications 1 à 6, **caractérisé en ce que** la caractéristique de l'acide nucléique est un type de nucléotide donné, choisi de préférence parmi l'un quelconque de A, T, U, G, C, au niveau d'une certaine position dans la molécule d'acide nucléique, de préférence la position étant au sein de 100 nucléotides de l'extrémité soit 5' soit 3' de la molécule d'acide nucléique, de façon spécialement préférée où les acides nucléiques sont choisis pour des nucléotides communs au sein des 10 nucléotides à côté de l'extrémité 5' et/ou 3', de préférence pour un ou plusieurs types de nucléotides terminaux en 5' et/ou 3' communs.

8. Procédé selon l'une quelconque des revendications 1 à 7, **caractérisé en ce que** ladite molécule d'ARN est un ARN pleine longueur et/ou la molécule d'acide nucléique séparée comprend la séquence de l'ADNc ou de l'ARN pleine longueur ou complète.

9. Procédé selon la revendication 2, **caractérisé en ce que** la détermination de la séquence comprend la détermination de la séquence d'au moins 5, de préférence d'au moins 8, nucléotides du fragment, en particulier de son extrémité soit 5' soit 3', encore plus préférentiellement la détermination de la séquence totale du fragment.

10. Procédé selon l'une quelconque des revendications 1 à 9, **caractérisé en ce que** les acides nucléiques sont divisés en sous-groupes où au moins 10 % de tous les sous-groupes comprennent la quantité moyenne des acides nucléiques de tous les sous-groupes +/- 50 %.

11. Procédé selon l'une quelconque des revendications 1 à 10, **caractérisé en ce que** les acides nucléiques sont divisés en sous-groupes où au moins 10 % des sous-groupes contiennent 2 acides nucléiques ou moins, de préférence 1 acide nucléique.

12. Procédé selon l'une quelconque des revendications 1 à 11, **caractérisé en ce que** la séparation des acides nucléiques comprend l'amplification spécifique des acides nucléiques à partir dudit groupe de matrices, de préférence où l'amplification est effectuée par extension nucléotidique à partir d'une amorce, de préférence par PCR, de façon particulièrement préférée où l'amplification est effectuée en utilisant des amorces qui sélectionnent au moins un, de préférence au moins deux, en particulier au moins deux adjacents, nucléotides différents d'après une partie d'amorce non spécifique, en vertu de quoi des molécules d'acide nucléique sont amplifiées, lesquelles comprennent le nucléotide sélectionné comme la caractéristique d'acide nucléique spécifique d'un sous-groupe.

13. Procédé selon l'une quelconque des revendications 1 à 12, **caractérisé par** la fixation aux fragments d'un marqueur spécifique du sous-groupe, de préférence où le marqueur spécifique du sous-groupe est un ou plusieurs nucléotides, qui sont de préférence co-déterminés durant la détermination de la séquence telle que définie dans la revendication 2.

14. Procédé selon l'une quelconque des revendications 1 à 13, comprenant en outre l'amplification des molécules d'acide nucléique, de préférence après la séparation, avant la détermination de la séquence, de façon particulièrement préférée où ladite amplification est effectuée par PCR et au moins une molécule de nucléotide est amplifiée jusqu'à la phase de saturation de la PCR, de façon particulièrement préférée au moins 10 % des différentes molécules de nucléotides sont amplifiées jusqu'à la phase de saturation de la PCR.

15. Procédé selon l'une quelconque des revendications 1 à 14, **caractérisé en ce que** les sous-groupes avec des molécules d'acide nucléique très abondantes sont exclus de la détermination de la séquence, où les sous-groupes avec des molécules d'acide nucléique très abondantes sont des sous-groupes comprenant plus de 1000 % de molécules d'acide nucléique au-dessus de la quantité moyenne de tous les sous-groupes.

16. Procédé selon l'une quelconque des revendications 1 à 15, **caractérisé en ce que** durant la séparation de l'acide nucléique, un brin sélectionné est séparé ou un brin sélectionné est marqué, où de préférence les fragments du brin sélectionné sont également marqués.


### SEQUENCE LISTING

<110> Lexogen GmbH
<120> RNA analytics method
<130> R 57882
<150> EP09178923.0
   <151> 2009-12-11
<160> 21
<170> PatentIn version 3.3
<210> 1
   <211> 55
   <212> DNA
   <213> Artificial
<220>
   <223> primer
<400> 1
   actgtaaaac gacggccagt atagttattg atatgtaata cgactcacta taggg 55
<210> 2
   <211> 54
   <212> DNA
   <213> Artificial
<220>
   <223> primer
<400> 2
   acggagccta tctatatgtt cttgacattt tttttttttt tttttttttt tttt 54
<210> 3
   <211> 29
   <212> DNA
   <213> Artificial
<220>
   <223> primer
<400> 3
   gttattgata tgtaatacga ctcactata 29
<210> 4
   <211> 31
   <212> DNA
   <213> Artificial
<220>
   <223> primer
<400> 4
   gacatttttt tttttttttt tttttttttt t 31
<210> 5
   <211> 22
   <212> DNA
   <213> Artificial
<220>
   <223> primer
<400> 5
   taatacgact cactataggg gg 22
<210> 6
   <211> 22
   <212> DNA
   <213> Artificial
<220>
   <223> primer
<400> 6
   taatacgact cactataggg ga 22
<210> 7
   <211> 22
   <212> DNA
   <213> Artificial
<220>
   <223> primer
<400> 7
   taatacgact cactataggg gc 22
<210> 8
   <211> 22
   <212> DNA
   <213> Artificial
<220>
   <223> primer
<400> 8
   taatacgact cactataggg gt 22
<210> 9
   <211> 26
   <212> DNA
   <213> Artificial
<220>
   <223> primer
<400> 9
   tttttttttt tttttttttt tttttc 26
<210> 10
   <211> 26
   <212> DNA
   <213> Artificial
<220>
   <223> primer
<400> 10
   tttttttttt tttttttttt tttttg 26
<210> 11
   <211> 26
   <212> DNA
   <213> Artificial
<220>
   <223> primer
<400> 11
   tttttttttt tttttttttt ttttta 26
<210> 12
   <211> 202
   <212> DNA
   <213> Artificial
<220>
   <223> random sequence
<400> 12
   cattacgtcc atatgagttc acggtccctt gaacttttat ggtaggtggt aggctcggcg 60
   aatctagctt tggagcttcg ccggactcaa caaggtaagg aggagcatcg ctctctcgac 120
   cactcaagac gggatattac ttgtgtcaag gagataatcg gaactattct ttagaatcca 180
   gctcgccgaa atcgtcaggc ga 202
<210> 13
   <211> 242
   <212> DNA
   <213> Artificial
<220>
   <223> random sequence
<400> 13
   ctagcttcgg tgtcatcccg gaaggcccac gttgtgcggc aatactagag ataaaagcgg 60
   caaagctaac accgaaagcc tatactggct acccgtctcg ttcggtggca ctaactagac 120
   tcctcatcag gcataggtga ccgctcgctc tcgtgccaag gtctcccgag acttccgaga 180
   tagtaataac tgaacatgga gaccggtatt gttaagctac atcaattgtg ggcgaaacga 240
   ac 242
<210> 14
   <211> 275
   <212> DNA
   <213> Artificial
<220>
   <223> random sequence
<400> 14
   cgttccggcc tgaagctcgg ggatccggcc cccccctaac ttcgctttct caaacgtaca 60
<210> 15
   <211> 297
   <212> DNA
   <213> Artificial
<220>
   <223> random sequence
<400> 15
   cagcccggac gcactgagat gatacgtgtt gaaccggcct tcactgtata ttatgctcac 60
   gagccctaga ttcatcaaaa aacaggtaca cttctcatcc tgactataca gctttcagtc 120
   atcctacgat gggaatctag agcccataga catatatgag cacactactc ttggtaacat 180
   ctcttgtcac atacattcgc caatctgaat ccttttctga cagccagttc tcatgatcca 240
   acacttaagg atttagcatt acggggcggg aggagaatcg aatactcgcc caccgtc 297
<210> 16
   <211> 304
   <212> DNA
   <213> Artificial
<220>
   <223> random sequence
<400> 16
   actggagagc accgaacata ctcctagccc gggatgacaa tgtcctaacg ccacccacta 60
   agggtaaggc tctaattgga aggtagtcca aatacgctcc atgacgagct tcgctctcaa 120
   ggctcgcagt cagaacgtat cgactatgcg actctaattc caaacccaga acctgagcga 180
   ggcagtcgtt agttaatgac gcttgccgag agaacagtaa aggagttctt cgatgaggta 240
   ctacgacatt cacatgtgtc atgggtcggt taagcatctg cgtgattgat tccggggggg 300
   tgtt 304
<210> 17
   <211> 297
   <212> DNA
   <213> Artificial
<220>
   <223> random sequence
<400> 17
   cagcaggtcg catatatcaa aagggaaagc cagctcgcct agacgtcgtt caatggtagg 60
   tactttaatt tttagagggg cttccccatg cttttggaga ttggcctatc ggtagtgagg 120
   ataccggcct ccacgctgcg tgatgagcac aatcattgtt ctcggagacg gaggacccgg 180
   aaggtaacga gcccaaaggt cattcatacc atatagggcg taacctcatt tagcgcgact 240
   gacgtgcaag gggcatccga cctgcgagga aggggccttg gctctgtagg atataat 297
<210> 18
   <211> 275
   <212> DNA
   <213> Artificial
<220>
   <223> random sequence
<400> 18
   tatcgaaagc cctaaggatt ttttttgggg aatcgattgt gttaagcagg gacggcttca 60
   aaattcgtct aataagattc tctggccatt accctaacag cgccatactc tatagacgca 120
   cgcctacctt aggcgcctcc cgtccccgga tccgagctcc caaaacccag cgacctcttc 180
   atgctaagga cttcatttgg acccgtcagg cactgctcca tgaagaacga catgaggatt 240
   tggagtatta aaggcttaac actgtagcgc caccg 275
<210> 19
   <211> 242
   <212> DNA
   <213> Artificial
<220>
   <223> random sequence
<400> 19
   gtgtcgtaac tgagcgatac agaacgacgc tgagtcatcg aggcaaatgc gtccacccgc 60
   acctgcgcat cccatacaag gtggcacaac ttagtaggac ttatatgcgg acttcaccgg 120
   tacgagaaga gttgaagact aaattatgac gtgacaaacg aaagagtaaa acaacatgcg 180
   tagctcttca tgaagcggca gagcaaacct tgattaaacc ccttgattgg caacactaca 240
   cg 242
<210> 20
   <211> 202
   <212> DNA
   <213> Artificial
<220>
   <223> random sequence
<400> 20
   cggttacccg gcgttaggcc tatgtaccgc ccgacgtact tgctaggggt catactaccg 60
   acgatccctg ctaacaaaga acagtaccgg ctttccttaa ctactcagtg ctactaaaac 120
   tagcatgagg gttgagatca tctcatccag ttgggtccag cgcatgatta attgctttac 180
   tcgcacttta attcggcttc ta 202
<210> 21
   <211> 160
   <212> DNA
   <213> Artificial
<220>
   <223> random sequence
<400> 21
   ggaggcacga cgagtatcta gtgtctgcac gggactccgg aggacattcc ctacaagtta 60
   ccggcgtcag tagcagcaag actggtctgt ctacccctgc ctgacaaagt ctttcttgga 120
   tttcggaccg aaactcggcc caacatgcca ttggccatat 160